# EUROPEAN PATENT APPLICATION

(11) **EP 2 311 494 A1**
(43) Date of publication of application: **20.04.2011**
(21) Application number: 09773511.2
(22) Date of filing: 01.07.2009
(51) Int. Cl.: A61K 45/00, A61K 47/48, A61P 3/10, A61P 9/10, A61P 11/02, A61P 17/00, A61P 17/06, A61P 17/16, A61P 25/00, A61P 25/18, A61P 25/20, A61P 25/24, A61P 25/28, A61P 27/02, A61P 27/06, A61P 35/00, A61P 43/00, C07D 403/14

(54) **HISTONE MODIFICATION INHIBITOR SPECIFIC TO TARGET GENE**

(30) Priority: 01.07.2008 JP 2008172795
(71) Applicant: Nihon University, Tokyo 102-8275 (JP)
(72) Inventor: NAGASE Hiroki, Tokyo 102-8275 (JP); SUGIYAMA Hiroshi, Kyoto-shi Kyoto 602-8435 (JP); SUZUKI Tukasa, Tokyo 102-8275 (JP); BANDO Toshikazu, Kyoto-shi Kyoto 606-8173 (JP); KIMURA Makoto, Tokyo 102-8275 (JP); OHTSUKI Akimichi, Kyoto-shi Kyoto 606-8266 (JP)
(74) Representative: Denison, Christopher Marcus
(86) International application number: PCT/JP2009/062054
(87) International publication number: WO 2010/001933

(57) **Abstract**

The present invention provides a target gene-specific histone modification regulator, which comprises a conjugate between a histone modification regulator and a polyamide capable of recognizing a regulatory region in a target gene. This enables the provision of a target gene-specific histone modification regulator.

## Description

### TECHNICAL FIELD

The present invention relates to histone modification regulators and so on.

### BACKGROUND ART

Histones are major proteins in eukaryotic chromosomes and can be divided into two major groups: nucleosomal histones and histone H1. Nucleosomal histones are subdivided into 4 classes: H2A, H2B, H3 and H4. H2A, H2B, H3 and H4 are elements constituting the histone core. The histone core has an octamer structure comprising two molecules of each of H2A, H2B, H3 and H4. The histone core having this octamer structure and one molecule of histone H1 are joined with DNA of about 200 base pairs to form a nucleosome, which is a basic structural unit of chromatin. The DNA of about 200 base pairs wraps around the histone core in 1.75 turns and further links nucleosomes together. The moiety used for linking nucleosomes is referred to as linker DNA. Histone H1 assembles such nucleosomes into a higher ordered structure. Histones are very rich in positively charged amino acids including lysine and arginine. Since DNA is negatively charged, positively charged histones strongly bind to DNA.

Histones are deeply involved in transcriptional regulation of genes in eukaryotic chromosomes. For example, once histones have undergone various modifications such as acetylation, phosphorylation, methylation and ubiquitination, a change will be induced in the binding state between DNA and histone or in the modified state of DNA, which in turn promotes or suppresses gene transcription. In view of this fact, some attempts have been made to regulate the transcription of disease-related genes (e.g., oncogenes and tumor suppressor genes) by using histone modification regulators to thereby treat cancers and other diseases.

For example, Patent Document 1 discloses the prevention and/or treatment of cancers with a histone deacetylase inhibitor (HDAC inhibitor), which is one of the histone modification regulators.

Patent Document 2 discloses the prevention and/or treatment of joint diseases with an HDAC inhibitor.

Non-patent Document 1 discloses possible implications of HDAC inhibitors for neurodegenerative diseases.

Patent Document 3 discloses that valproic acid (VPA), which is one of the HDAC1 inhibitors, and analogs thereof are used as tranquilizers or antiepileptics. In addition, valproic acid (VPA) and analogs thereof are currently undergoing clinical trials for approval as anti-HIV (AIDS) drugs (Patent Document 4).

Moreover, suberoylanilide hydroxamic acid (SAHA), which is one of the HDAC inhibitors, is commercially available under the name Vorinostat from Merck & Co., Inc., and is used as a therapeutic agent for cutaneous T cell lymphoma (CTCL) (Patent Document 5).

With respect to histone methylation, for example, AMI-1, which is an inhibitor of histone arginine methyltransferase PRMT, is reported to regulate the expression of intranuclear receptor genes in cancer cells without substantially affecting histone lysine methylation (Non-patent Document 2).

Furthermore, it is reported that histone arginine methylation contributes to the pluripotency of stem cells (Non-patent Document 3), and histone arginine methyltransferase inhibitors can also be used to elucidate the mechanism of stem cell pluripotency (Patent Document 6).

### Prior Art Documents

### Patent Documents

[Patent Document 1] JP 2008-505970 A
[Patent Document 2] JP 2006-335666 A
[Patent Document 3] WO 1999/066920
[Patent Document 4] WO 2007/121429
[Patent Document 5] WO 2008/002914
[Patent Document 6] WO 2008/017024

### Non-patent Documents

[Non-patent Document 1] Hahnen E, Hauke J, Trankle C, Eyupoglu IY, Wirth B, Blumcke I (2008), Histone deacetylase inhibitors: possible implications for neurodegenerative disorders. Expert Opin Investig Drugs 17(2):169-84
[Non-patent Document 2] Donghang Cheng, Neelu Yadav, Randall W. King, Maurice S. Swanson, Edward J. Weinstein_, and Mark T. Bedford (2004) Small Molecule Regulators of Protein Arginine Methyltransferases JBC 279(23):23892-23899
[Non-patent Document 3] Maria-Elena Torres-Padillal, David-Emlyn Parfittl, Tony Kouzaridesl & Magdalena Zernicka-Goetz (2007) Histone arginine methylation regulates pluripotency in the early mouse embryo Nature 445, 214-218

### DISCLOSURE OF THE INVENTION

### PROBLEMS TO BE SOLVED BY THE INVENTION

Under these circumstances, there has been a demand for target gene-specific histone modification regulators.

### MEANS FOR SOLVING THE PROBLEMS

As a result of extensive and intensive efforts made to solve the above problems, the inventors have found that it was possible to synthesize a conjugate between a histone modification regulator and a polyamide capable of recognizing a regulatory region in a target gene, and that this conjugate has the ability to control the expression of the target gene and to regulate histone modification at a specific site on the genome. The present invention has been completed on the basis of these findings.

Namely, the present invention provides the following target gene-specific histone modification regulators and so on.
(1) A target gene-specific histone modification regulator, which comprises a conjugate between a histone modification regulator and a polyamide capable of recognizing a regulatory region in a target gene.
(2) The regulator according to (1) above, wherein the histone modification regulator regulates at least one histone modification selected from the group consisting of acetylation, phosphorylation, methylation, ubiquitination, sumoylation, proline isomerization, deimination and ADP ribosylation in a genomic site-specific manner.
(3) The regulator according to (2) above, wherein the histone modification regulator is a histone deacetylase inhibitor.
(4) The regulator according to (3) above, wherein the histone deacetylase inhibitor is a hydroxamic derivative, a cyclic tetrapeptide, a benzamide derivative, or an aliphatic acid.
(5) The regulator according to (4) above, wherein the hydroxamic derivative is suberoylanilide hydroxamic acid, suberoyl bis-hydroxamic acid or trichostatin A.
(6) The regulator according to (4) above, wherein the cyclic tetrapeptide is trapoxin A or trapoxin B.
(7) The regulator according to (4) above, wherein the benzamide derivative is MS-275.
(8) The regulator according to (4) above, wherein the aliphatic acid is butyrate or NaB.
(9) The regulator according to (2) above, wherein the histone modification regulator is a histone methyltransferase inhibitor.
(10) The regulator according to (9) above, wherein the histone methyltransferase inhibitor is a compound having an aromatic ring, a compound having a heterocyclic ring, or a derivative thereof.
(11) The regulator according to (10) above, wherein the compound having an aromatic ring, the compound having a heterocyclic ring, or a derivative thereof is a compound having a naphthalene compound as its skeletal structure, a compound having an azo compound as its skeletal structure, or a derivative thereof.
(12) The regulator according to (11) above, wherein the compound having a naphthalene compound as its skeletal structure is AMI-1, suramin or 7-amino-4-hydroxy-2-naphthalenesulfonic acid (J acid).
(13) The regulator according to (11) above, wherein the compound having an azo compound as its skeletal structure is Direct Yellow 26, Direct Yellow 50, or Direct Red 75.
(14) The regulator according to any one of (1) to (13) above, wherein the target gene is a gene involved in cell regulation.
(15) The regulator according to (14) above, wherein the gene involved in cell regulation is an oncogene or a tumor suppressor gene.
(16) The regulator according to (14) above, wherein the gene involved in cell regulation is a gene involved in the maintenance and/or differentiation of stem cells or progenitor cells.
(17) The regulator according to (15) above, wherein the oncogene is a gene for Cytoplasmic tyrosine kinase, a gene for regulatory GTPase, a gene for tyrosine kinase receptor, a gene for intracellular serine or threonine kinase or a regulatory subunit thereof, a gene for an adaptor protein in the signal transduction system, or a gene for transcription factor.
(18) The regulator according to (15) above, wherein the tumor suppressor gene is the p16INK4a (CDKN2A) gene, p21 (CDKN1A) gene, APC gene, RASSF1 gene, RB gene, NF1 gene, NF2 gene, p19 (CDKN2D) gene, WT1 gene, VHL gene, BRCA1 gene, BRCA2 gene, CHEK2 gene, Maspin gene, p73 gene, DPC4 (SMAD4) gene, MSH2 gene, MLH1 gene, PMS2 gene, DCC gene, PTEN gene, p57KIP2 (CDKN1C) gene, PTC gene, TSC1 gene, TSC2 gene, EXT1 gene, EXT2 gene, or p53 gene.
(19) The regulator according to (16) above, wherein the gene involved in the maintenance and/or differentiation of stem cells or progenitor cells is the LIF (leukaemia inhibitory factor) gene, OCT3/4 gene, NANOG gene, SOX2 gene, KLF4 gene, MYC gene, MYCN gene, or p16INK4a (CDKN2A) gene.
(20) The regulator according to (1) above, wherein the conjugate is represented by the following formula (1), (2), (3), (4) or (5). 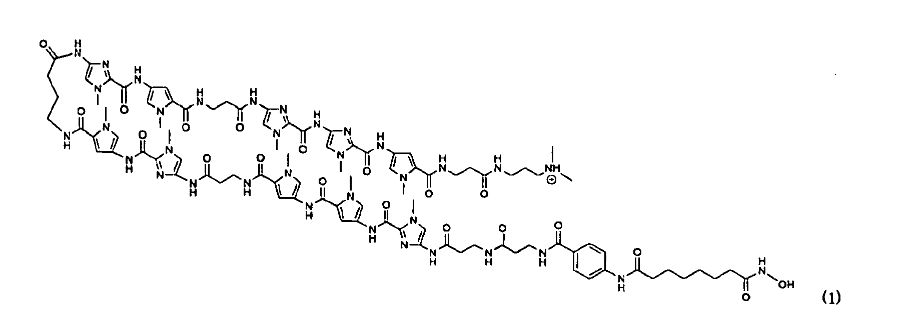 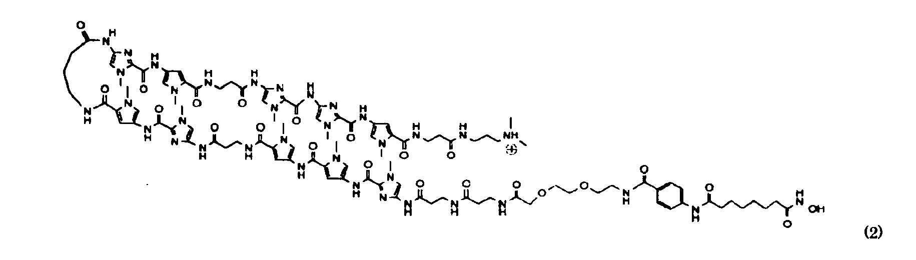 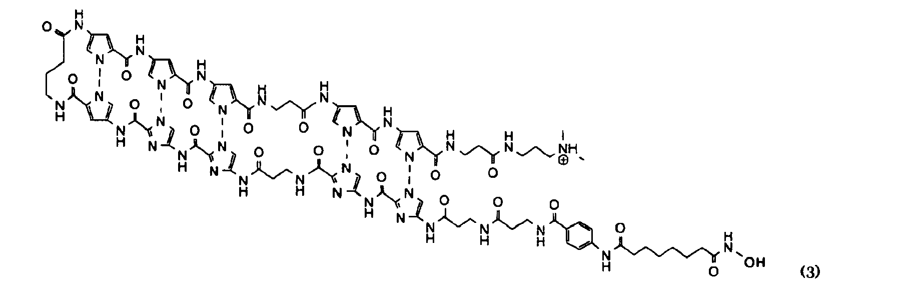 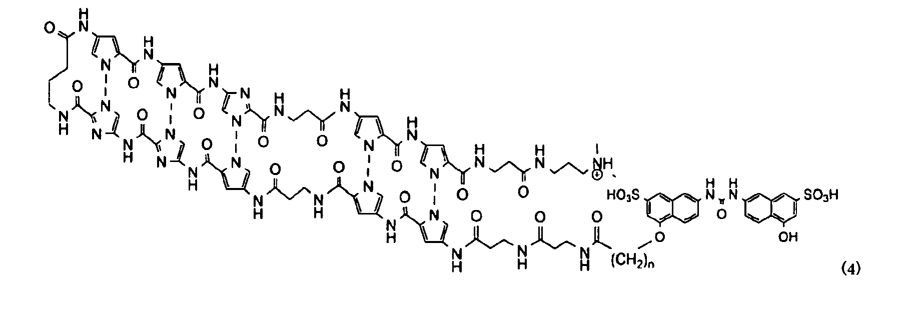 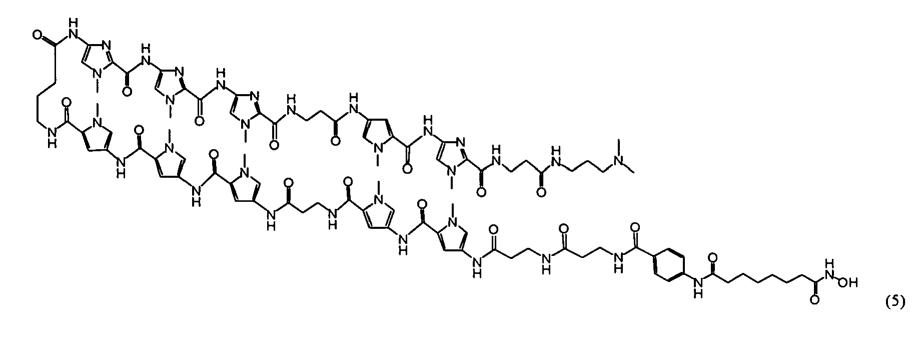
(21) A method for target gene-specific regulation of histone modification, which uses a conjugate between a histone modification regulator and a polyamide capable of recognizing a regulatory region in a target gene.
(22) The method according to (21) above, wherein the histone modification regulator regulates at least one histone modification selected from the group consisting of acetylation, phosphorylation, methylation, ubiquitination, sumoylation, proline isomerization, deimination and ADP ribosylation in a genomic site-specific manner.
(23) The method according to (22) above, wherein the histone modification regulator is a histone deacetylase inhibitor.
(24) The method according to (23) above, wherein the histone deacetylase inhibitor is a hydroxamic derivative, a cyclic tetrapeptide, a benzamide derivative, or an aliphatic acid.
(25) The method according to (24) above, wherein the hydroxamic derivative is suberoylanilide hydroxamic acid, suberoyl bis-hydroxamic acid or trichostatin A.
(26) The method according to (24) above, wherein the cyclic tetrapeptide is trapoxin A or trapoxin B.
(27) The method according to (24) above, wherein the benzamide derivative is MS-275.
(28) The method according to (24) above, wherein the aliphatic acid is butyrate or NaB.
(29) The method according to (22) above, wherein the histone modification regulator is a histone methyltransferase inhibitor.
(30) The method according to (29) above, wherein the histone methyltransferase inhibitor is a compound having an aromatic ring, a compound having a heterocyclic ring, or a derivative thereof.
(31) The method according to (30) above, wherein the compound having an aromatic ring, the compound having a heterocyclic ring, or a derivative thereof is a compound having a naphthalene compound as its skeletal structure, a compound having an azo compound as its skeletal structure, or a derivative thereof.
(32) The method according to (31) above, wherein the compound having a naphthalene compound as its skeletal structure is AMI-1, suramin or 7-amino-4-hydroxy-2-naphthalenesulfonic acid (J acid).
(33) The method according to (31) above, wherein the compound having an azo compound as its skeletal structure is Direct Yellow 26, Direct Yellow 50, or Direct Red 75.
(34) The method according to any one of (21) to (33) above, wherein the target gene is a gene involved in cell regulation.
(35) The method according to (34) above, wherein the gene involved in cell regulation is an oncogene or a tumor suppressor gene.
(36) The method according to (34) above, wherein the gene involved in cell regulation is a gene involved in the maintenance and/or differentiation of stem cells or progenitor cells.
(37) The method according to (35) above, wherein the oncogene is a gene for Cytoplasmic tyrosine kinase, a gene for regulatory GTPase, a gene for tyrosine kinase receptor, a gene for intracellular serine or threonine kinase or a regulatory subunit thereof, a gene for an adaptor protein in the signal transduction system, or a gene for transcription factor.
(38) The method according to (35) above, wherein the tumor suppressor gene is the p16INK4a (CDKN2A) gene, p21 (CDKN1A) gene, APC gene, RASSF1 gene, RB gene, NF1 gene, NF2 gene, p19 (CDKN2D) gene, WT1 gene, VHL gene, BRCA1 gene, BRCA2 gene, CHEK2 gene, Maspin gene, p73 gene, DPC4 (SMAD4) gene, MSH2 gene, MLH1 gene, PMS2 gene, DCC gene, PTEN gene, p57KIP2 (CDKN1C) gene, PTC gene, TSC1 gene, TSC2 gene, EXT1 gene, EXT2 gene, or p53 gene.
(39) The method according to (36) above, wherein the gene involved in the maintenance and/or differentiation of stem cells or progenitor cells is the LIF (leukaemia inhibitory factor) gene, OCT3/4 gene, NANOG gene, SOX2 gene, KLF4 gene, MYC gene, MYCN gene, or p16INK4a (CDKN2A) gene.
(40) The method according to (21) above, wherein the conjugate is represented by the following formula (1), (2), (3), (4) or (5). 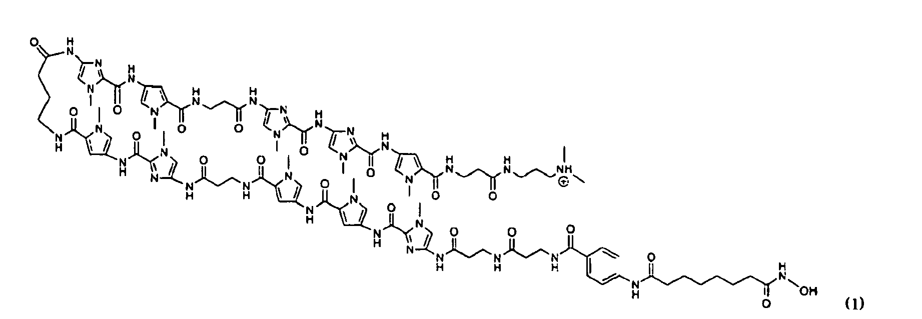 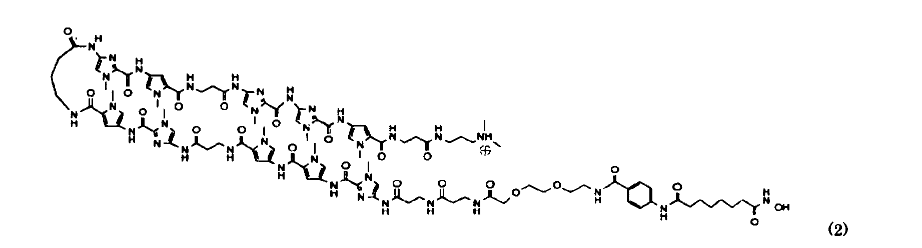 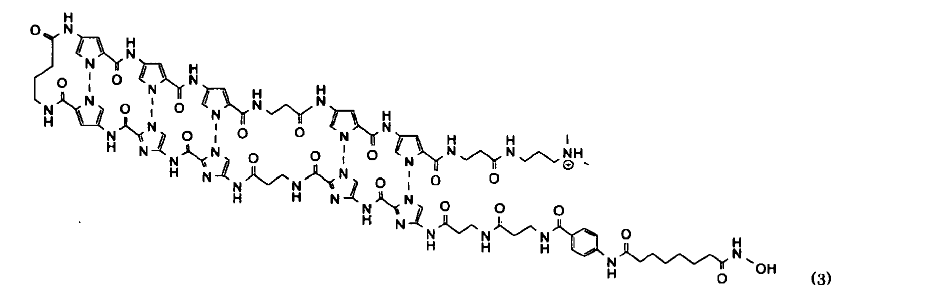 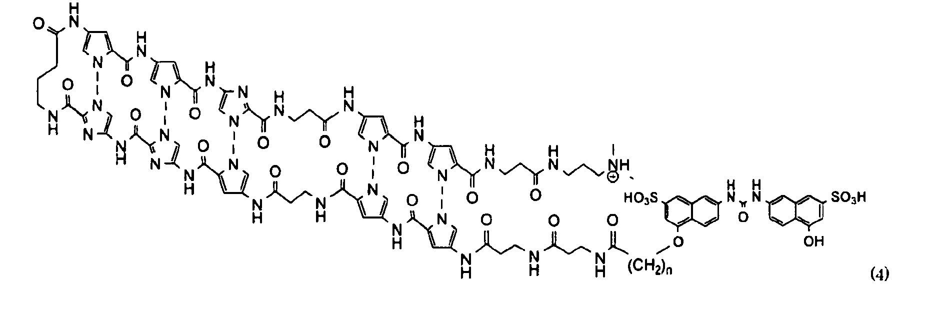 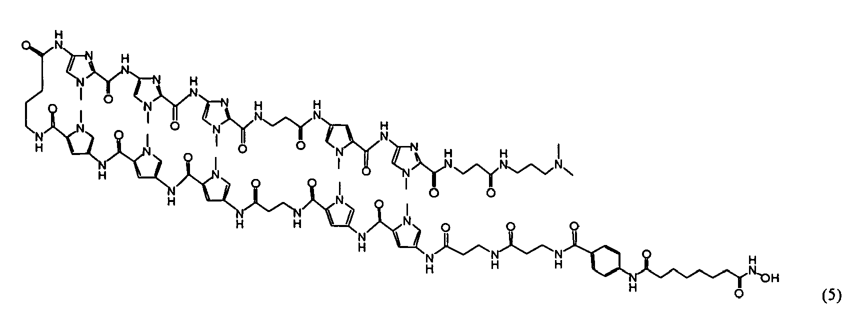
(41) A pharmaceutical composition, which comprises the conjugate according to any one of(1)to (20) above.
(42) The pharmaceutical composition according to (41) above, which is used for cancer treatment.
(43) A method for cancer treatment, which comprises administering the conjugate according to any one of(1)to (20) above.
(44) A reagent for cell function study, which comprises the conjugate according to any one of(1)to (20) above.

### ADVANTAGEOUS EFFECT OF THE INVENTION

The present invention provides target gene-specific histone modification regulators and so on. According to a preferred embodiment of the present invention, the regulators can be used for prevention and/or treatment of cancers and other diseases.

### BRIEF DESCRIPTION OF THE DRAWINGS

Figure 1 shows the sequence of the promoter region in the p16 gene.
Figure 2 is a graph showing the results of HDAC activity assay.
Figure 3 is a graph showing the results of cell proliferation assay on HeLa cells.
Figure 4 is a graph showing the results of cell proliferation assay on 4 cancer cell lines.
Figure 5 shows photographs of HeLa cell line observed under an optical microscope (left: before cell proliferation assay, right: after cell proliferation assay).
Figure 6 is a graph showing the results of real-time RT-PCR.
Figure 7 is a graph showing the results of Western blotting.
Figure 8 shows the sequence of the promoter region in the p53 gene.
Figure 9 shows the sequence of the promoter region in the MYCN gene.
Figure 10 is a graph showing the results analyzed for genomic region-specific histone acetylation.
Figure 11 is a graph showing the results analyzed for LARP1 gene expression by quantitative PCR.
Figure 12 is a graph showing the results of proliferation assay on breast cancer cells.

### Sequence Listing Free Text

SEQ ID NO: 2: synthetic DNA,
SEQ ID NO: 3: synthetic DNA,
SEQ ID NO: 4: synthetic DNA,
SEQ ID NO: 5: synthetic DNA,
SEQ ID NO: 8: synthetic DNA,
SEQ ID NO: 9: synthetic DNA,
SEQ ID NO: 10: synthetic DNA,
SEQ ID NO: 11: synthetic DNA,
SEQ ID NO: 12: synthetic DNA,
SEQ ID NO: 13: synthetic DNA.

### MODES FOR CARRYING OUT THE INVENTION

The present invention will be described in more detail below.

It should be noted that all documents cited herein, including prior art documents, patent gazettes and other patent documents, are incorporated herein by reference. Moreover, this specification incorporates the contents of the specification, claims and/or drawings disclosed in Japanese Patent Application No. 2008-172795, based on which the present application claims priority.

### 1. Summary of the present invention

As described above, histones are deeply involved in transcriptional regulation of genes in eukaryotic chromosomes. Once histones have undergone modifications, a change will be induced in the binding state between DNA and histone or in the modified state of DNA, which in turn promotes or suppresses gene transcription. Examples of histone modifications include histone acetylation. In cells, histone acetylation is known to be regulated by the action of histone acetyltransferase (HAT) and histone deacetylase (HDAC). In recent years, some members of HAT and HDAC have been identified. This histone acetylation allows histone proteins to have negatively charged acetyl groups, whereby histones are charged negatively to induce dissociation between histones and between DNA and histone. This fact suggests that histone acetylation is deeply involved in transcriptional regulation of genes. For example, once histone acetylation has proceeded, the condensed nucleosomes will be relaxed to induce DNA demethylation. Once relaxation of the condensed nucleosomes has occurred in gene regulatory regions and DNA demethylation has proceeded, transcription factors can be bound to these regulatory regions to thereby promote gene expression. In view of the foregoing, if demethylation can be induced, for example, in the regulatory region of the tumor suppressor gene p16 through induction of histone acetylation, p16 expression is enhanced to allow prevention and/or treatment of cancers. For use in this purpose, HDAC inhibitors are known as reagents capable of inducing histone acetylation. HDAC inhibitors inhibit the action of intracellular HDAC to thereby induce histone acetylation. However, conventional HDAC inhibitors act randomly in cells and hence have problems in that they allow little expression of a target gene, or rather, more strongly induce the expression of non-target genes.

In contrast, the present invention relates to target-specific histone modification regulators and so on, which allow target-specific regulation of histone modifications. More specifically, the target-specific histone modification regulators of the present invention comprise a conjugate between a histone modification regulator and a polyamide capable of recognizing a regulatory region in a target gene. Examples of such a histone modification regulator in the conjugate include histone deacetylase inhibitors (HDAC inhibitors) and its analogs. Likewise, examples of such a polyamide include pyrrole-imidazole amide (PIP) and its analogs.

The present invention has been completed on the basis of the findings that it was possible to synthesize a conjugate between a histone modification regulator and a polyamide capable of recognizing a regulatory region in a target gene, and that this conjugate acted on histone modifications, which are involved in target gene expression, in a genomic site-specific manner to thereby regulate target gene expression.

For example, if a polyamide capable of recognizing a regulatory region in the tumor suppressor gene p16 is conjugated with an HDAC inhibitor, when a target-specific histone modification regulator comprising this conjugate is applied to cancer cells, the polyamide in the HDAC inhibitor conjugate would bind to the HDAC enzyme's active genomic site in a target-specific manner, and the HDAC inhibitor in the conjugate acts on histone modifications. This action on histone modifications affects the gene regulatory region in the tumor suppressor gene p16, which in turn induces binding of transcription factors to the gene regulatory region to up-regulate transcription, thus promoting p16 expression. Further, promoted p16 expression allows suppression of cancer cell proliferation and/or control of aging signals toward stem cells and other cells (WO2004/026109). Moreover, since histone deacetylation is inhibited in a target-specific manner, the conjugate can minimize the problem of more strongly inducing the expression of non-target genes, and hence has fewer side effects, etc.

### 2. Conjugate

The conjugate of the present invention has a histone modification regulator and a polyamide capable of recognizing a regulatory region in a target gene.

Preferred examples of such a histone modification regulator constituting the conjugate are those regulating at least one histone modification selected from the group consisting of acetylation, phosphorylation, methylation, ubiquitination, sumoylation, proline isomerization, deimination and ADP ribosylation, or those inhibiting or activating histone chaperones that act on nucleosomes. More preferred histone modification regulators are those regulating histone acetylation, phosphorylation or methylation, and even more preferred are those regulating histone acetylation or methylation. Once histones have undergone various modifications such as acetylation, phosphorylation, methylation and ubiquitination, a change will be induced in the binding state between DNA and histone or in the modified state of DNA, which in turn promotes or suppresses gene transcription. In view of this fact, histone modification regulators can be used to regulate the transcription of disease-related genes (e.g., oncogenes and tumor suppressor genes) to thereby prevent and/or treat cancers and other diseases.

For example, histone acetylation allows the condensed nucleosomes to be relaxed to induce DNA demethylation. Once DNA demethylation has proceeded in gene regulatory regions, transcription factors can be bound to these regulatory regions to thereby promote gene expression. On the other hand, histone deacetylation induces nucleosomal condensation and DNA methylation. Once DNA methylation has proceeded in gene regulatory regions, transcription factors cannot be bound to these regulatory regions to thereby suppress gene expression. Thus, a conjugate capable of recognizing a regulatory region in a tumor suppressor gene, according to one embodiment of the present invention, may be applied to cancer cells to induce histone acetylation in a genomic site-specific manner and thus induce demethylation of the regulatory region in the tumor suppressor gene to thereby enhance the expression of the tumor suppressor gene, or alternatively, a conjugate capable of recognizing a regulatory region in an oncogene, according to another embodiment of the present invention, may be applied to cancer cells to suppress histone acetylation in a genomic site-specific manner and thus induce methylation of the regulatory region in the oncogene to thereby suppress the expression of the oncogene. In these cases, cancers can be prevented and/or treated.

Histone phosphorylation would exert various functions depending on the site where phosphorylation occurs, and there are many types of kinases that regulate histone phosphorylation. For example, phosphorylation of H2A appears to be involved in reactions against DNA damage, while phosphorylation of histone H2B and histone H3 (particularly serine 10 and 28) appears to be involved in the expression of c-fos or other genes and in the condensation of chromosomes during cell division or apoptosis. Thus, when a conjugate according to yet another embodiment of the present invention is applied to cancer cells to cause phosphorylation in a genomic site-specific manner (e.g., at serine 10 in histone H3), the cell cycle of the cancer cells can be regulated and their proliferation can be arrested, whereby cancers can be prevented and/or treated. Further, in the pineal body, phosphorylation of serine 10 in histone H3 is suggested to regulate arylalkylamine-N-acetyltransferase gene expression and contribute to animal's circadian activity (Endocrinology 148_(4):1465-1472 (2007)). Thus, when a conjugate according to yet another embodiment of the present invention is applied, it is expected to induce phosphorylation/dephosphorylation of histone H3 in a genomic site-specific manner and is also expected for use in the elucidation of human's circadian rhythm or the alleviation of insomnia and other diseases. Moreover, in some gene regions, histone phosphorylation induces histone acetylation and affects gene expression in cells, cell activity or cell proliferation. For example, when histone H3 serine 10 is phosphorylated in the histone deacetylase HDAC 1 gene region, 14-3-3 protein binds to the phosphorylated histone H3 and further induces acetylation of histone H3 lysine 9 and 14 to enhance HDAC1 gene expression (EMBO J. 27(1): 88-99 (2007)).

Histone methylation appears to cause activation of transcription or silencing of gene loci, depending on the position of methylation (methylation sites and the number thereof), and is involved in epigenetic regulation of gene expression. For example, it is known that methylation of histone H3 lysine 4 up-regulates gene expression, while trimethylation of histone H3 lysine 9, 27 and 79 causes up-regulation upon addition of a single methyl group and causes down-regulation upon addition of three methyl groups. Methylated histone H3 binds to heterochromatin protein 1 (HP1), and the presence or absence of this binding regulates the chromatin structure and gene expression. Phosphorylation of histone H3 serine 10 described above appears to be catalyzed by AURKB (aurora kinase B) and is reported to suppress methylation of the adjacent lysine 9. Such interaction between phosphorylation and methylation of histone H3 has been elucidated to regulate the chromatin structure and gene expression, and is expected for use in suppression of cancer cell proliferation. It is suggested that phosphorylation of histone H4 serine 1 contributes to spermatogenesis.

Histone ubiquitination is reported to occur in H2A, H2B and H3. Although detailed functions remain unknown, it is suggested that H2A ubiquitination contributes to suppression of gene expression (Nature 2004 431 873-878), for example, based on the finding that H2A histone ubiquitination activating enzyme is contained in the Polycomb complex causing suppression of gene transcription. It is suggested that H2B ubiquitination regulates histone methylation, and thus, appears to regulate gene expression.

Sumoylation of histone occurs at specific residues in histone H4, H2A and H2B, and the same lysine residues as those where acetylation or ubiquitination occurs are also modified with SUMO. Sumoylation appears to suppress acetylation or ubiquitination because high-molecular-weight ubiquitin-like proteins occupy the histone tail moiety and thereby prevent other histone-modifying enzymes from reaching the histone tail. This suggests that sumoylation contributes to regulation of gene expression.

Proline isomerization is reported to be induced at histone H3 proline 38 by the action of an enzyme called FPR4. This H3P38 isomerization would cause a structural change in the histone H3 tail to induce H3K36 methylation by JMJD2 or other histone methyltransferases, as a result of which gene expression is regulated.

Deimination is a reaction in which arginine residues in histones H3 and H4 are converted into citrulline residues by the action of PADI4 or other enzymes. This reaction inhibits arginine methylation and prevents gene expression mediated by histone arginine methylation, thus suggesting that deimination acts to suppress gene expression.

ADP ribosylation is regulated by enzymes called MART (mono(ADP-ribosyl)transferase) and PART (poly(ADP-ribosyl)transferase). ADP ribosylation appears to be involved in repair of genetic damage because these enzymes are expressed upon DNA duplex damage.

DNA wounded on histone proteins further forms a nucleosome structure to prevent genetic information from being read out. In contrast, when genetic information on DNA is required, various proteins act on nucleosomes to release DNA from histones. Such proteins are referred to as histone chaperones, and TFIID, NAP1 and TAFIβ are known as histone chaperones. Histone chaperones contribute to the formation and disruption of nucleosomes.

Although there is some uncertainty about the detailed regulatory mechanism of histone modification-induced formation and disruption of the nucleosome structure, it has been gradually elucidated that histone modifications contribute to gene expression, transcription factors, cell division, meiotic division, cell death, chromosome replication, and gene repair, etc. Histone modifications appear to be involved in various clinical conditions because of their contribution to gene regulation and various cellular regulatory mechanisms, as described above. Histone modifications have been shown to contribute to the above-mentioned carcinogenesis, brain and nervous diseases (e.g., autism, bipolar disorder, schizophrenia, epilepsy), as well as the onset of various lifestyle-related diseases and stem cell-based regenerative medicine. When histone modification is regulated in a manner specific to a gene site, such regulation allows detailed analysis of cell functions and can also be used as a reagent for mechanism elucidation and therapy development for intractable diseases.

Enzymes catalyzing histone acetylation are HATs, while enzymes catalyzing histone deacetylation are HDACs. HDACs can be divided into three classes (classes I to III), based on the findings obtained from structural analysis of yeast HDACs. As members of class I, HDAC 1, HDAC2, HDAC3, HDAC8 and HDAC11 are known, and their expression can be found everywhere. As members of class II, HDAC4, HDAC5, HDAC7 and HDAC9 are known for class IIa, and HDAC6 and HDAC 10 are known for class IIb. Likewise, as members of class III, SIRT1 to SIRT7 are known. Since these HDACs are present in abundance in cells, the genome is generally regulated in the direction of deacetylation. And in turn, deacetylation of the genome causes condensation of nucleosomes. Moreover, the genome will optionally be acetylated by HAT. Upon acetylation of the genome, condensed nucleosomes would be relaxed to allow proteins such as DNA-binding proteins to contact with DNA or histones. Histone acetylation may be regulated, e.g. when these enzymes including HATs or HDACs are inhibited to induce histone acetylation or histone deacetylation. Substances used to induce histone acetylation include HDAC inhibitors or those which indirectly induce histone acetylation through inhibition of DNA methylation, i.e., hydralazine, 5-aza-2'-deoxycytidine, zebularine, or magnesium valporate. Preferred are HDAC inhibitors. Examples of HDAC inhibitors include hydroxamic derivatives, cyclic tetrapeptides, benzamide derivatives or aliphatic acids. Preferred are hydroxamic acid derivatives. Examples of hydroxamic acid derivatives include suberoylanilide hydroxamic acid (SAHA), suberoyl bis-hydroxamic acid, or trichostatin A. Examples of cyclic tetrapeptides include trapoxin A (TPX) or trapoxin B. Examples of benzamide derivatives include MS-275. Examples of aliphatic acids include butyrate or NaB. On the other hand, substances used to induce histone deacetylation include H3-CoA-20 or Lys-CoA.

Such histone acetylation regulators can be synthesized in a known manner or are commercially available. For example, SAHA is available as vorinostat (Zolinza) from Merck & Co., Inc. TPX is available from Novartis, Basal, Switzerland. MS-275 is available from ALEXIS Biochemicals (Lausen, Switzerland). NaB is available from Biomol (Hamburg, Germany).

Enzymes catalyzing histone phosphorylation are kinases, while enzymes catalyzing histone dephosphorylation are phosphatases. Histone phosphorylation or histone dephosphorylation may be induced, e.g., when these enzymes including kinases or phosphatases are inhibited. Substances known to induce histone phosphorylation include histone acetyltransferase inhibitors or Cantharidin. On the other hand, substances known to induce histone dephosphorylation include kinase inhibitors such as aurora kinase inhibitors. Examples of known aurora kinase inhibitors include 4-(4'-benzamidoanilino)-6,7-dimethoxyquinazoline or VX-680.

Such histone phosphorylation regulators can be synthesized in a known manner or are commercially available. For example, 4-(4'-benzamidoanilino)-6,7-dimethoxyquinazoline is available from Merk Biosciences. VX-680 is available from Vertex.

Enzymes catalyzing histone methylation are histone methyltransferases, while enzymes catalyzing histone demethylation are histone demethylases. Enzymes known as histone methyltransferases include lysine methyltransferase and arginine methyltransferase. Examples of known histone demethylases include JHMD1, JHMD2A, GASC1, JMJD2A, JMJD2B, JMJD2C, and JMJD2D. Histone methylation may be regulated, e.g. when these histone methyltransferases or histone demethylases are inhibited to induce histone methylation or histone demethylation. Substances used to induce histone methylation include MAO inhibitors such as Phenelzine. On the other hand, substances used to induce histone demethylation include histone methyltransferase inhibitors, histone demethylases and activators thereof, as well as histone citrullination enzymes. Preferred are histone methyltransferase inhibitors. Examples of histone methyltransferase inhibitors (e.g., Chaetocin inhibitors, lysine methyltransferase (LSD) inhibitors, or protein arginine methyltransferase (PRMT) inhibitors) include compounds having an aromatic ring, compounds having a heterocyclic ring, or derivatives thereof. Such compounds having an aromatic ring or a heterocyclic ring, or derivatives thereof may be, for example, those having a naphthalene compound or an azo compound as their skeletal structure, or derivatives thereof. Examples of compounds having a naphthalene compound as their skeletal structure include AMI-1, suramin or 7-amino-4-hydroxy-2-naphthalenesulfonic acid (J acid). Examples of compounds having an azo compound as their skeletal structure include Direct Yellow 26, Direct Yellow 50, or Direct Red 75. Further examples of compounds having an aromatic ring include tranylcypromine, which is an LSD inhibitor. AMI-1 can also be presented as an example of PRMT inhibitors.

Such histone methylation regulators can be synthesized in a known manner or are commercially available. For example, Pargyline (tranylcypromine) is available from GlaxoSmithKline. AMI-1 is available from Merk & Co., Inc. Suramin is available from Sigma-Aldrich. Phenelzine is available from Spectrum Chemicals.

Enzymes catalyzing histone ubiquitination are ubiquitin-activating enzymes, ubiquitin-conjugating enzymes, or ubiquitin transferases (ubiquitin ligases), while enzymes catalyzing histone deubiquitination are deubiquitinating enzymes (DUBs). Histone ubiquitination may be regulated, e.g. when ubiquitin-activating enzymes, ubiquitin-conjugating enzymes, ubiquitin transferases, or deubiquitinating enzymes are inhibited to induce histone ubiquitination or histone deubiquitination. Substances used to induce histone ubiquitination include iodoacetamide. On the other hand, substances used to induce histone deubiquitination include UBEI-41.

Such histone ubiquitination regulators can be synthesized in a known manner or are commercially available. For example, iodoacetamide is available from Sigma-Aldrich. UBEI-41 is available from Biogenova Corp.

Substances used to induce sumoylation of histone include arsenic trioxide (As₂O₃), while substances used to induce desumoylation of histone include lactacystin.

Such regulators for sumoylation of histone can be synthesized in a known manner or are commercially available. For example, As₂O₃ and lactacystin are available from Sigma-Aldrich.

Substances used to inhibit histone deimination include PAD4 inhibitors, which inhibit deimination of arginines in histones (see WO2007/056389 and WO2007/0518).

Such histone deimination regulators can be synthesized in a known manner or are commercially available.

Substances used to inhibit ADP ribosylation of histone include diadenosine 5',5"-pl,p4-tetraphosphate (Ap4A) (Molecular and Cellular Biochemistry 74:17-20 (1987)).

Such regulators for ADP ribosylation of histone can be synthesized in a known manner or are commercially available. For example, procedures for Ap4A synthesis can be found in Biophosphates and Their Analogues-Synthesis, Structure, Metal and Activity, eds. Bruzik, K. S. & Stec, W. J. (Elsevier, Amsterdam), pp. 451-464. (1986).

Substances used to inhibit proline isomerization of histone include Juglone and derivatives thereof.

Such regulators for proline isomerization of histone can be synthesized in a known manner or are commercially available. For example, Juglone is available from Sigma-Aldrich.

Substances used to inhibit histone chaperones that act on nucleosomes include NSC348884, which is a nucleophosmin inhibitor.

Those inhibiting or activating histone chaperones that act on nucleosomes can be synthesized in a known manner or are commercially available. For example, NSC348884 is available from NCI (NCI, Maybridge, LeadQuest and the Available Chemical Database).

The polyamide (target-recognizing polyamide), which is a component of the conjugate, is designed to recognize a regulatory region in a target gene. In the case of using conventional histone modification regulators comprising no target-recognizing polyamide, histone modification is randomly regulated in cells, as a result of which only non-target genes are regulated in their expression and target gene expression cannot be regulated in some cases. Since the conjugate of the present invention comprises a target-recognizing polyamide, it achieves more specific regulation of target gene expression than conventional regulators alone. As used herein, the phrase "recognize(ing) a regulatory region" is intended to mean that a target-recognizing polyamide binds to a regulatory region and/or the proximity thereof in a target gene (e.g., through hydrogen bonding or crosslinkage formation (crosslinking)). DNA has an alternating sequence of core histone-binding sites of 146 nucleotides and non-core histone binding sites of about 50 nucleotides (linker histone-binding and non-binding sites), and it forms a nucleosome structure approximately every 200 nucleotides. Linker histone-binding sites and core histone-binding sites are regions whose DNA is difficult to be recognized by DNA-binding proteins. For this reason, these linker histone-binding sites and core histone-binding sites are regions resistant to nucleases or bisulfite treatment. In a preferred embodiment of the present invention, a region highly susceptible to bisulfite treatment (i.e., a region having no histone protein bound thereto) is searched in a gene regulatory region whose DNA is methylated (generally having a nucleosome structure), and a target-recognizing polyamide is designed to bind (e.g., through hydrogen bonding or crosslinkage formation (crosslinking)) to such a region highly susceptible to bisulfite treatment located near the regulatory region (i.e., a susceptible region located within 200 nucleotides (corresponding to a single nucleosome), preferably 100 nucleotides from the regulatory region). The "regulatory region" recognized by such a target-recognizing polyamide may be, for example, a promoter, an enhancer, a repressor or an insulator, preferably a promoter. The gene regulatory region and/or the proximity thereof, to which the target-recognizing polyamide binds, is preferably linker DNA (i.e., a non-histone binding DNA region in nucleosomes). Examples of such a target-recognizing polyamide include pyrrole-imidazole polyamide (PIP), or bridged nucleic acids/locked nucleic acids (LNAs).

PIP is a polyamide containing N-methylpyrrole units (Py), N-methylimidazole units (Im) and a γ-aminobutyric acid moiety, in which Py, Im and the γ-aminobutyric acid moiety are linked to each other through amide bonds (-C(=O)-NH-) (Trauger et al, Nature, 382, 559-61(1996); White et al, Chem Biol., 4, 569-78(1997); and Dervan, Bioorg Med Chem., 9, 2215-35(2001)). PIP is folded as a whole into a U-shaped conformation where the γ-aminobutyric acid moiety serves as a linker (γ**-**linker). In this U-shaped conformation, two chains each containing Py and Im are located in parallel, sandwiching the linker. When pairs of Py and Im between these two chains are specific combinations (Py/Im pair, Im/Py pair, Py/Py pair, or Im/Im pair), they can bind to specific base pairs in DNA with high affinity. For example, the Py/Im pair can bind to a C-G base pair, while the Im/Py pair can bind to a G-C base pair. Moreover, the Py/Py pair can bind to both A-T and T-A base pairs (White et al, Chem Biol., 4, 569-78(1997); Dervan: Bioorg Med Chem., 9, 2215-35(2001)). PIP may further contain 3-hydroxypyrrole (Hp) or β-alanine. With respect to Hp, the Hp/Py pair can bind to a T-A base pair (White at al, Nature, 391, 468-71(1998)). With respect to β-alanine/β-alanine, it can bind to a T-A or A-T base pair. It is therefore possible to design PIP capable of recognizing a regulatory region in a target gene, for example, when pair combinations of Py and Im are changed in line with a target DNA sequence. Procedures for design and preparation of PIP are known (Japanese Patent No. 3045706, JP 2001-136974 A and WO03/000683). More specifically, PIP is designed and synthesized as follows to recognize a regulatory region in a target gene. Namely, detailed procedures for design of PIP recognizing the promoter region of the p16 gene are described later in the Example section.

Design of PIP recognizing a non-histone and non-transcription factor binding site located near the promoter region of the p53 gene is described in more detail in the design examples given later. Design of PIP recognizing a non-histone and non-transcription factor binding site located near the promoter region of the MYCN gene is also described in more detail in the design examples given later. PIPs thus designed may be prepared in a simple manner, for example, by automated synthesis based on the solid-phase method with Fmoc (9-fluorenylmethoxycarbonyl) (i.e., solid-phase Fmoc method). According to the solid-phase Fmoc method, for example, a conjugate between PIP and a labeling substance such as FITC (fluorescein isothiocyanate) may also be synthesized. The resulting conjugate can be used to confirm that PIP recognizes a specific DNA sequence.

Bridged nucleic acids/locked nucleic acids (LNAs) are designed to recognize a regulatory region in a target gene and can be synthesized as 2',4'-BNAs having a methylene bridge between 2'-oxygen and 4'-carbon atoms in RNA or as 2',4'-ENAs (ethylene-bridged nucleic acids) having an ethylene bridge between 2'-oxygen and 4'-carbon atoms in RNA. LNAs are also available from Proligo.

The conjugate of the present invention can be synthesized, for example, by attaching the above histone modification regulator to the above target-recognizing polyamide. Attachment between the above histone modification regulator and the above target-recognizing polyamide may be accomplished in a known manner (J. Am. Chem. SOC. 1995, 117, 2479-2490).

Such "attachment" may be accomplished either directly or via a linker. Any linker may be used for this purpose as long as it does not block the action of the histone modification regulator and it does not prevent the target-recognizing polyamide from recognizing a gene region. Examples include an amide linkage, a phosphodisulfide linkage, an ester linkage, a coordinate linkage, an ether linkage and so on.

The conjugate of the present invention obtained as described above may be exemplified by the following.
<p16 Gene-specific conjugate (1), (2) and (5), p53 gene-specific conjugate (3), as well as MYCN gene-specific conjugate (4)>

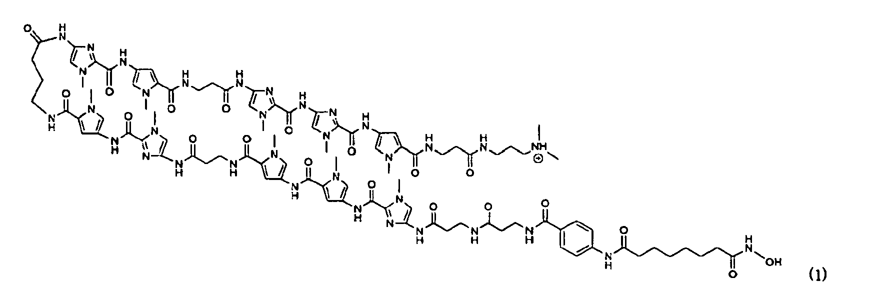

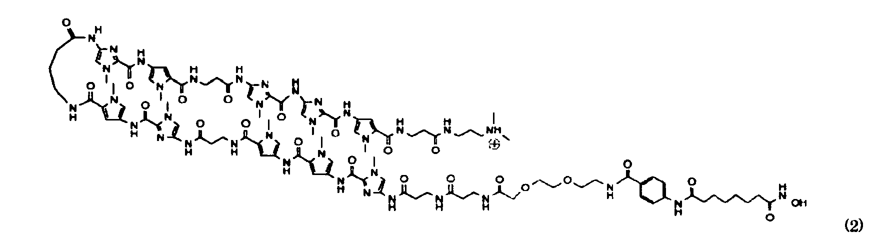

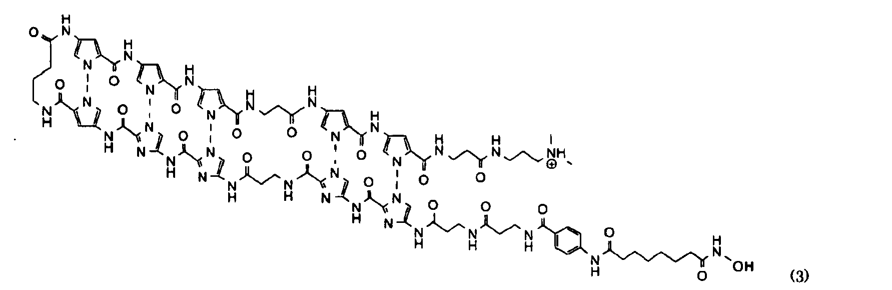

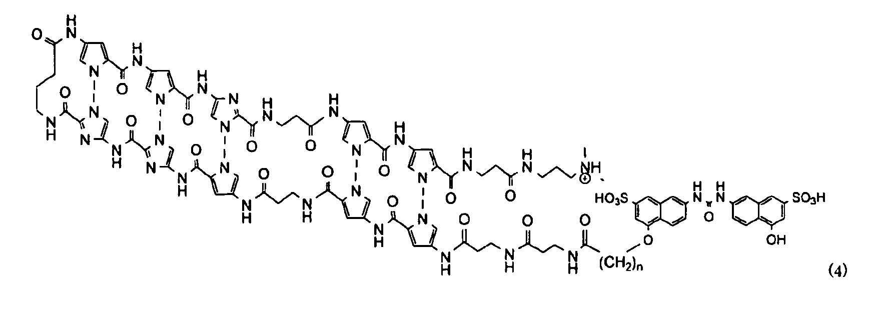

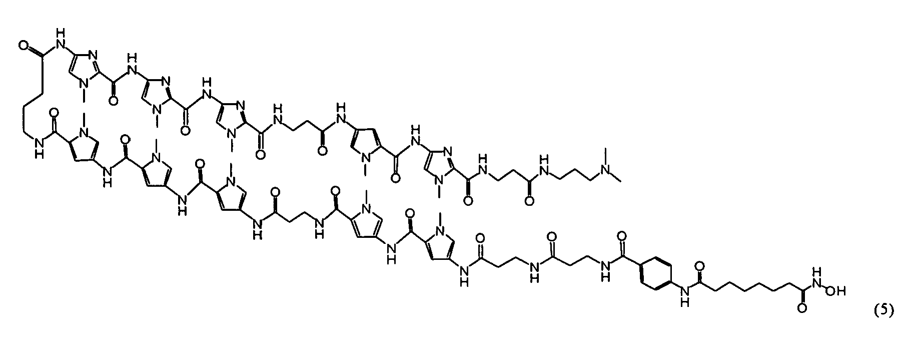

### 3. Target gene-specific histone modifier

The target gene-specific histone modifier of the present invention comprises the above conjgate of the present invention. Likewise, the method of the present invention for target gene-specific histone modification is a method using the above conjugate of the present invention. As used herein, the phrase "target gene-specific" is intended to mean being specific to one or more target genes (Humans are known to have about 100000 genes in their genomic region, and in sequences involved in gene expression, a single transcription factor is known to recognize a specific sequence and regulate its expression in several thousands to several hundreds of regions. For this reason, for example, the phrase "target gene-specific" is intended to mean being specific to one to several thousands (e.g., 1 to 5000, 1 to 4000, 1 to 3000, 1 to 2000 or 1 to 1000), one to several hundreds (e.g., 1 to 500, 1 to 400, 1 to 300, 1 to 200 or 1 to 100), or 1 to 50, preferably 1 to 10, more preferably 1 to 5 (5, 4, 3, 2 or 1) genes selected from those mentioned above). For example, some transcription factors regulate the expression of several hundreds to several thousands of genes by their recognition sequences of 5 nucleotides and thereby control biological events (e.g., the MYC gene has 3000 to 4000 downstream genes). Thus, even when the target gene-specific histone modifier of the present invention recognizes a sequence with low specificity (i.e., a sequence common to several thousands to several hundreds of genes), the histone modifier of the present invention controls biological events and may be effective for disease treatment or regeneration therapy, etc. It should be noted that the target gene-specific histone modifier of the present invention can reduce the number of its target genes, for example, by increasing the number of nucleotides in a sequence recognized by the polyamide or by selecting a sequence with higher specificity as a sequence recognized by the polyamide.

The phrase "using the conjugate of the present invention" is intended to include, for example, in vitro use of the conjugate of the present invention by being contacted with cells in vitro, as well as administration (in vivo use) to mammals (e.g., non-human mammals) or laboratory organisms (e.g., non-human organisms), etc. The term "contact" is intended to mean that the conjugate of the present invention and cells are allowed to exist in the same reaction system or culture system, for example, by adding the conjugate of the present invention to a cell culture vessel, by mixing the cells with the conjugate of the present invention, or by culturing the cells in the presence of the conjugate of the present invention.

Examples of the target gene intended in the modifier or modification method of the present invention include genes involved in cell regulation. Such genes involved in cell regulation include at least one gene selected from the group consisting of tumor suppressor genes, oncogenes, neuronal regulator-encoding genes, and genes involved in the maintenance and/or differentiation of stem cells or progenitor cells. Preferred is at least one gene selected from the group consisting of tumor suppressor genes, oncogenes, and genes involved in the maintenance and/or differentiation of stem cells or progenitor cells.

Examples of tumor suppressor genes include p16INK4a (CDKN2A) gene, p21 (CDKN1A) gene, APC gene, RASSF1 gene, RB gene, NF1 gene, NF2 gene, p19 (CDKN2D) gene, WT1 gene, VHL gene, BRCA1 gene, BRCA2 gene, CHEK2 gene, Maspin gene, p73 gene, DPC4 (SMAD4) gene, MSH2 gene, MLH1 gene, PMS2 gene, DCC gene, PTEN gene, p57KIP2 (CDKN1C) gene, PTC gene, TSC1 gene, TSC2 gene, EXT1 gene, EXT2 gene, or p53 gene. Preferred tumor suppressor genes include p16 gene, p21 gene, APC gene, RASSF1 gene, RB gene, or p53 gene.

Examples of oncogenes include genes for Cytoplasmic tyrosine kinase (e.g., Src-family, Syk-ZAP-70 family, or BTK family), genes for regulatory GTPase (e.g., ras gene (e.g., HRas gene)), genes for tyrosine kinase receptor (e.g., EGF receptor (EGFR) gene, PDGFR gene or VEGFR gene), genes for intracellular serine or threonine kinase or a regulatory subunit thereof (e.g., RAF1 gene or aurora kinase gene), genes for adaptor proteins in the signal transduction system (e.g., GRB2 gene or SHC gene), or genes for transcription factors (e.g., MYC (c-Myc) gene or MYCN gene). Preferred oncogenes include MYCN gene, c-Myc gene, aurora kinase gene, ras gene, or EGFR gene.

Examples of neuronal regulator-encoding genes include SHC3 gene, NMDA receptor genes (e.g., NR2A gene or NR2C gene), or Dopamine receptor genes (e.g., DRD1 or DRD2).

Examples of genes involved in the maintenance and/or differentiation of stem cells or progenitor cells include the LIF (leukaemia inhibitory factor) gene, OCT3/4 gene, NANOG gene, SOX2 gene, KLF4 gene, MYC gene, MYCN gene, or p16INK4a (CDKN2A) gene, and preferred examples include the MYC gene, NANOG gene, OCT3/4 gene, or KLF4 gene. In another preferred embodiment of the present invention, genes involved in the maintenance and/or differentiation of stem cells or progenitor cells include MYC gene, SOX2 gene, OCT3/4 gene, or KLF4 gene.

In a case where the target gene intended in the modifier or modification method of the present invention is a tumor suppressor gene or an oncogene, the modifier or modification method of the present invention can be used, for example, to prevent and/or treat cancers.

In a case where the target gene intended in the modifier or modification method of the present invention is a neuronal regulator-encoding gene, the modifier or modification method of the present invention can be used, for example, to treat neurological diseases.

In a case where the target gene intended in the modifier or modification method of the present invention is a gene involved in the maintenance and/or differentiation of stem cells or progenitor cells, the modifier or modification method of the present invention can be used, for example, to create iPS cells.

Moreover, the modifier of the present invention may further comprise carriers and/or additives in addition to the conjugate of the present invention, depending on the intended purpose. Furthermore, in the modification method of the present invention, the conjugate may be used in combination with carriers and/or additives, depending on the intended purpose. Examples of such carriers and additives include water, acetic acid, organic solvents, collagen, polyvinyl alcohol, polyvinylpyrrolidone, carboxyvinyl polymer, carboxymethylcellulose sodium, sodium polyacrylate, sodium alginate, water-soluble dextran, carboxymethyl starch sodium, pectin, methylcellulose, ethylcellulose, xanthan gum, gum arabic, casein, agar, polyethylene glycol, diglycerine, glycerine, propylene glycol, petrolatum, paraffin, stearyl alcohol, stearic acid, human serum albumin, mannitol, sorbitol, lactose, surfactants and so on.

The amount of the conjugate of the present invention to be used in the modifier and modification method of the present invention will vary depending on the intended purpose. Those skilled in the art would be able to select an appropriate amount for the conjugate of the present invention, depending on the intended purpose.

The present invention also encompasses the use of the above conjugate of the present invention for the manufacture of the target gene-specific histone modifier of the present invention. The present invention further encompasses the above conjugate of the present invention for use in target gene-specific histone modification.

Moreover, the present invention encompasses a kit for target gene-specific regulation of histone modification, which comprises the conjugate of the present invention. The kit of the present invention may further comprise carriers and/or additives as described above for the modifier, in addition to the conjugate of the present invention. Furthermore, the kit of the present invention may also comprise auxiliaries, a container(s) used for this purpose, other necessary accessories, manufacturer's instructions and so on, if necessary.

Further, the present invention provides a reagent for cell function study, which comprises the conjugate of the present invention. The method of the present invention for cell function study is a method using the above conjugate of the present invention. As used herein, the phrase "using the conjugate of the present invention" is intended to include, for example, in vitro use of the conjugate of the present invention by being contacted with cells in vitro, as well as administration (in vivo use) to mammals (e.g., non-human mammals such as rats, rabbits, sheep, pigs, cattle, cats, dogs, monkeys) or laboratory organisms (e.g., non-human organisms such as Drosophila, nematode, E. coli, yeast, Xenopus laevis, Oryzias latipes, Salmo trutta, globefish, Arabidopsis thaliana, rice). The term "contact" is intended to mean that the conjugate of the present invention and cells are allowed to exist in the same reaction system or culture system, for example, by adding the conjugate of the present invention to a cell culture vessel, by mixing the cells with the conjugate of the present invention, or by culturing the cells in the presence of the conjugate of the present invention. In a certain embodiment of the present invention, when histone modification is regulated in a manner specific to a gene site by the conjugate of the present invention, such regulation allows detailed analysis of cell functions and is useful for mechanism elucidation or therapy development for intractable diseases. The reagent and/or method of the present invention for use in study can be targeted for mammals (e.g., non-human mammals such as rats, rabbits, sheep, pigs, cattle, cats, dogs, monkeys) or other laboratory organisms (e.g., non-human organisms such as Drosophila, nematode, E. coli, yeast, Xenopus laevis, Oryzias latipes, Salmo trutta, globefish, Arabidopsis thaliana, rice), etc. The reagent and/or method of the present invention for use in study may further comprise carriers and/or additives as described above for the modifier, in addition to the conjugate of the present invention. The amount of the conjugate of the present invention to be used in the reagent and/or method of the present invention for use in study will vary depending on the intended purpose. Those skilled in the art would be able to select an appropriate amount for the conjugate of the present invention, depending on the intended purpose.

### 4. Pharmaceutical composition

The pharmaceutical composition of the present invention comprises the above conjugate of the present invention. This pharmaceutical composition can be administered in vivo to prevent and/or treat various diseases. Diseases targeted by the pharmaceutical composition of the present invention include cancers, neurological/mental diseases, lifestyle-related diseases, sleep disorders, dermatologic, ophthalmologic or otorhinologic diseases and infections with strong local symptoms, allergic diseases, cellular aging-related diseases, resistance to thyroid hormone, aging, cystic fibrosis, as well as digestive system diseases. Among these target diseases, examples of cancers include brain tumor, cervical cancer, esophageal cancer, tongue cancer, lung cancer, breast cancer, pancreatic cancer, gastric cancer, small intestine or duodenal cancer, large bowel cancer (colon cancer, rectal cancer), bladder cancer, kidney cancer, liver cancer, prostate cancer, uterine cancer, ovarian cancer, thyroid cancer, gallbladder cancer, pharyngeal cancer, sarcomas (e.g., osteosarcoma, chondrosarcoma, Kaposi's sarcoma, myosarcoma, angiosarcoma, fibrosarcoma), leukemias (e.g., chronic myelogenous leukemia (CML), acute myelogenous leukemia (AML), chronic lymphocytic leukemia (CLL) and acute lymphocytic leukemia (ALL), lymphoma, multiple myeloma (MM)), pediatric solid tumors (e.g., neuroblastoma, hepatoblastoma, nephroblastoma, Ewing's sarcoma), retinoblastoma and melanoma, etc. Lifestyle-related diseases are not limited in any way and examples include hypertension, diabetes, etc. Examples of dermatologic, ophthalmologic or otorhinologic diseases and infections with strong local symptoms include psoriasis, chronic dermatitis, sinusitis, glaucoma, retinal degeneration, etc. Examples of allergic diseases include atopic dermatitis, pollinosis, etc. Examples of cellular aging-related diseases include skin wrinkles, sagging skin, pigmentation, etc. Examples of neurological/mental diseases include mania, depression, schizophrenia, autism, bipolar disorders, Alzheimer's disease, sleep disorders, dementia, etc.

Cancers can be prevented and/or treated when histone acetylation and/or methylation is regulated by the conjugate of the present invention to induce the expression of tumor suppressor genes (e.g., p16 gene, p21 gene, APC1 gene, RASSF1 gene, RB gene, or p53 gene) or to suppress the expression of oncogenes (e.g., MYCN gene, MYC gene, aurora kinase gene, ras gene, or EGFR gene).

Neurodegenerative diseases can be prevented and/or treated when histone acetylation and/or methylation is regulated by the conjugate of the present invention to induce the expression of GDNF or neurturin gene or to suppress the expression of APP (amyloid precursor protein) gene.

Cystic fibrosis and digestive system diseases can be prevented and/or treated when histone acetylation and/or methylation is regulated by the conjugate of the present invention to induce or suppress the expression of CFTR gene.

The pharmaceutical composition of the present invention may be in either oral or parenteral dosage form.

These dosage forms can be formulated in a routine manner, and may comprise pharmaceutically acceptable carriers and/or additives. Such carriers and additives include water, acetic acid, pharmaceutically acceptable organic solvents, collagen, polyvinyl alcohol, polyvinylpyrrolidone, carboxyvinyl polymer, carboxymethylcellulose sodium, sodium polyacrylate, sodium alginate, water-soluble dextran, carboxymethyl starch sodium, pectin, methylcellulose, ethylcellulose, xanthan gum, gum arabic, casein, agar, polyethylene glycol, diglycerine, glycerine, propylene glycol, petrolatum, paraffin, stearyl alcohol, stearic acid, human serum albumin, mannitol, sorbitol, lactose, and surfactants acceptable as pharmaceutical additives.

These additives are selected alone or in combination from among those listed above, as appropriate for the intended dosage form of the pharmaceutical composition of the present invention. Possible dosage forms for oral administration include tablets, capsules, fine granules, powders, granules, solutions, syrups, sprays, liniments, eye drops, external preparations, or other appropriate dosage forms. Possible dosage forms for parenteral administration include injectable dosage forms or the like. Injectable dosage forms may be administered systemically or locally, for example, by intravenous injection (e.g., drip infusion), subcutaneous injection, intratumoral injection, etc.

For example, for use as injectable formulations, the pharmaceutical composition of the present invention may be dissolved in a solvent (e.g., physiological saline, buffer, glucose solution, 0.1% acetic acid) and supplemented with appropriate additives (e.g., human serum albumin). Alternatively, the pharmaceutical composition of the present invention may be lyophilized for use as dosage forms that are reconstituted before use. As excipients for lyophilization, sugar alcohols and sugars (e.g., mannitol, glucose) may be used.

The dose of the pharmaceutical composition of the present invention or the conjugate of the present invention will vary depending on the age, sex and symptoms of a patient, the route of administration, the frequency of administration, and the intended dosage form. For example, in the case of adults (60 kg), the daily dose is 0.01 to 1000 mg, preferably 0.1 to 100 mg, and more preferably 1 to 30 mg. The mode of administration is selected as appropriate for the age and symptoms of a patient. The pharmaceutical composition of the present invention or the conjugate of the present invention may be administered, for example, as a single dose or in 2 to 4 divided doses per day.

Moreover, the present invention also encompasses the use of the above conjugate of the present invention for the manufacture of a pharmaceutical composition for cancer treatment. The present invention further encompasses a method for cancer treatment, which comprises administering the above conjugate of the present invention. The pharmaceutical composition and/or treatment method of the present invention can be targeted for mammals (e.g., humans, rats, rabbits, sheep, pigs, cattle, cats, dogs, monkeys). In some embodiments of the present invention, humans may be excluded from the target of the pharmaceutical composition and/or treatment method.

Moreover, the present invention also encompasses a kit for cancer treatment, which comprises the conjugate of the present invention. The kit of the present invention may further comprise pharmaceutically acceptable carriers and/or additives as described above for the pharmaceutical composition, in addition to the conjugate of the present invention. Furthermore, the kit of the present invention may also comprise auxiliaries, a container(s) used for this purpose, other necessary accessories, manufacturer's instructions and so on, if necessary.

The present invention will be further described in more detail by way of the following illustrative examples, which are not intended to limit the scope of the invention.

### EXAMPLES

### 1. Summary:

p16 is a tumor suppressor having the ability to arrest cell cycle through inhibition of the cell cycle regulator CDK. In cancer cells, DNA of the regulatory region in the p16 gene is methylated to suppress the expression of p16 gene. In this Example section, a compound (conjugate) was prepared by attaching SAHA to PIP capable of recognizing the regulatory region of the p16 gene. When this conjugate was used in HDAC activity assay, cell proliferation assay or the like, the synthesized conjugate was found to induce p16 gene expression in cancer cells and was also found to have an inhibitory effect on cancer cell proliferation. It should be noted that although SAHA is reported to induce histone acetylation and hence demethylation in the gene regulatory region, and is also reported to have an anticancer effect, it is generally known that SAHA does not cause re-expression of the p 16 gene.

### 2. Design and synthesis of conjugates and so on:

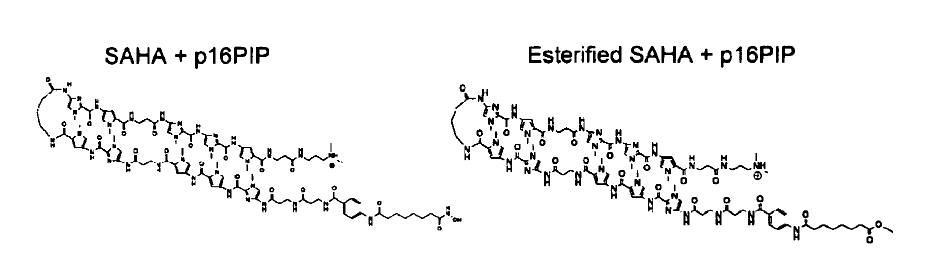

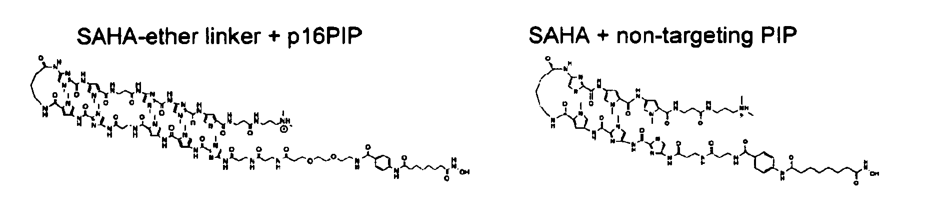

The four conjugates shown above (SAHA+p16 PIP, SAHA-ether linker+p16 PIP, esterified SAHA+p16 PIP and SAHA+non-targeting PIP) were designed and synthesized. SAHA+p16 PIP is a conjugate between PIP recognizing the promoter region of the p16 gene (p16 PIP) and SAHA. SAHA-ether linker+p16 PIP is a conjugate formed by linking p16 PIP and SAHA via an ether linker. Esterified SAHA+p16 PIP is a conjugate containing p16 PIP and esterified SAHA. Esterified SAHA has no anti-HDAC activity. SAHA+non-targeting PIP is a conjugate between PIP not recognizing the promoter region of the p16 gene (non-targeting PIP) and SAHA.

The above four conjugates were designed as follows.

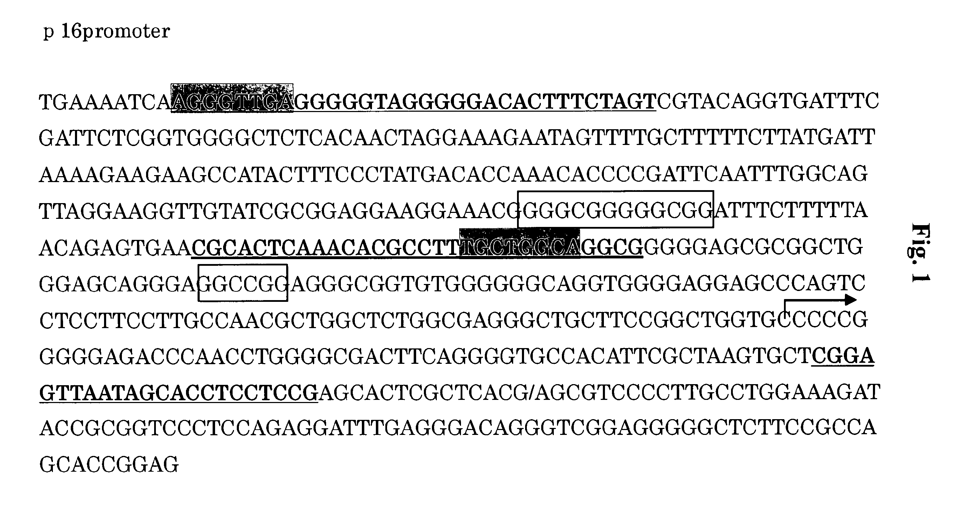

The sequence CGCACTCAAACACGCCTTTGCTGGCAGGCG located near the GC boxes in the promoter region of the p16 gene was used as a target of PI polyamide synthesis. Namely, a polyamide was designed to recognize the underlined sequence TGCTGGCA (-436 to -429) in the above sequence. Since the p16 gene is a tumor suppressor gene, the promoter region of the p16 gene is frequently methylated in cancer cells. In this case where the promoter region of the p16 gene is frequently methylated, a nucleosome structure is observed in the promoter site, to which histone proteins are bound. In this state, histones are deacetylated and DNA is condensed to generate a state where transcription factors cannot bind to the promoter region, thereby suppressing transcription of the p 16 gene. The binding state of histones in the promoter region of the p16 gene can be identified by M.SssI footprinting (Nucleic Acids Research, 2005, Vol. 33, No. 20 e176). Figure 1 shows the sequence of the promoter region (-450 to +110) in the p 16 gene (SEQ ID NO: 1). In the sequence shown in Figure 1, the sequence indicated with bold underline is regarded as a linker moiety which does not bind to histone proteins in cancer cells. In such a linker moiety, PI polyamide can bind to DNA, but the polyamide is highly likely not to bind to DNA in histone-binding regions because histone proteins are already bound to DNA in these regions. Moreover, since a GC box is regarded as a region used for transcription factor binding, the linker sequence CGCACTCAAACACGCCTTTGCTGGCAGGCG located near the two boxed GC boxes in the sequence shown in Figure 1 appears to be located near the transcription factor-binding region. Thus, if histone acetylation can be induced in this region, the distance between histones will widen to facilitate binding of transcription factors to the promoter. For this reason, the middle shadowed sequence TGCTGGCA in Figure 1 was used as a target and the polyamide was designed to recognize this sequence. A conjugate between such a polyamide recognizing this sequence and an HDAC inhibitor would be expected to induce histone acetylation in a target-specific manner and more effectively cause a change in the nucleosome structure. Such a change in the nucleosome structure would make it possible to cause re-expression of the p16 tumor suppressor gene in cancer cells. Moreover, it was further expected that re-expression of the p16 tumor suppressor gene allowed suppression of cell proliferation and induction of aging-induced apoptosis in cancer cells. The structural formula of SAHA+p16 PIP thus designed is shown below.

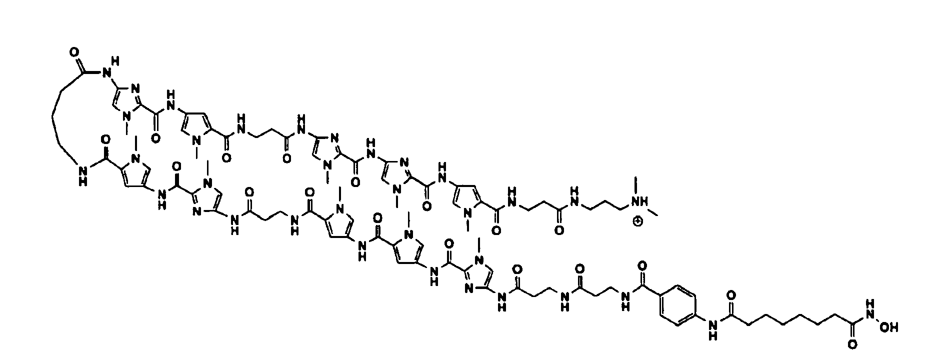

Non-targeting PIP was designed not to recognize the sequence of the above non-histone binding linker region in the p16 promoter. More specifically, non-targeting PIP was designed to recognize WGWCC. The underlined linker region in the promoter shown in Figure 1 does not contain this recognition sequence.

The above four conjugates were synthesized as follows.

It should be noted that the analysis instruments used in the synthesis described below are as follows.

### Analysis instruments

| | |
|---|---|
| NMR spectrometer: | JEOL JNM-AL400, |
| ESI-MS: | PESCIEX API165, |
| UV-Vis spectrometer: | Nano Drop ND-1000 Spectrophotometer, |
| HPLC unit: | JASCO PU-2080 Plus, |
| HPLC detector: | JASCO 807-IT, |
| HPLC column: | Chemcobond 5-ODS-H column (4.6 × 150 mm) (for analysis purposes), |
| | Chemcobond 5-ODS-H column (10 × 150 mm) (for preparative purposes), |
| Column chromatograph: | Merck Silica gel 60, |
| TLC plate: | Merck Silica gel 60 F₂₅₄. |

### Methyl 4-aminobenzoate 1:

Methyl 4-aminobenzoate 1 was purchased from Sigma-Aldrich.

### Synthesis of methyl 4-(8-methoxy-8-oxooctanamido)benzoate 2:

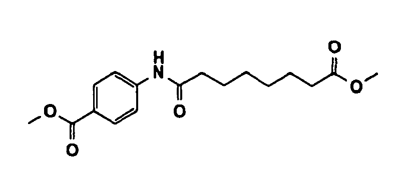

A 30 ml recovery flask was charged with methyl 4-aminobenzoate 1 (1.2 g, 8.0 mmol) and fully degassed and purged with Ar using a three-way stopcock. Then, dehydrated THF (15 ml) was added as a solvent using a syringe to dissolve the reagent, followed by addition of DIEA (2.1 ml) as a base. After ice cooling, methyl 8-chloro-8-oxooctanoate (1.2 ml, 8.2 mmol) was added. The mixture was stirred for 8 hours while gradually warming to room temperature. The reaction was monitored by TLC (10% MeOH/AcOEt) and HPLC (0.1% AcOH-CH₃CN, 0-100%, 20 min, 254 nm). After confirming that methyl 4-aminobenzoate was completely consumed, H₂O (10 ml) was added to quench the reaction. After addition of CH₂Cl₂ (25 ml), the organic layer was washed three times with saturated aqueous NaHCO₃ (30 ml) and then three times with 10% aqueous citric acid (30 ml). The collected organic layer was dried over MgSO₄ and subjected to suction filtration to remove solids. After collection of the organic layer, the solvent was distilled off under reduced pressure conditions and the residue was dried to give 2 as a white solid.

Yield: 2.4 g (94%),¹H NMR (CDCl₃, 400 MHz, δ): 7.93(d, J= 8.4 Hz, 2 H; CH), 7.53(d, J = 8.4 Hz, 2 H; CH), 7.31(brs, 1 H; NH), 3.83(s, 3 H; CH₃), 3.60(s, 3 H; CH₃), 2.39-2.22(m, 4 H; CH₂), 1.72-1.61(m, 2 H; CH₂), 1.59-1.55(m, 2 H; CH₂), 1.37-1.30(m, 4 H; CH₂).

### Synthesis of methyl 4-(8-(hydroxyamino)-8-oxooctanamido)benzoate 3:

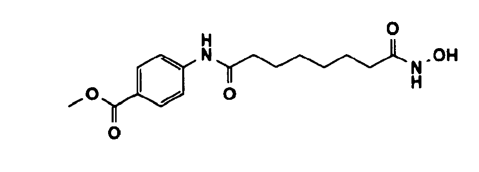

To a 20 ml recovery flask, methyl 4-(8-methoxy-8-oxooctanamido)benzoate 2 (212.3 mg, 0.68 mmol) was added and dissolved in a mixed solvent of THF (4 ml) and DMF (4 ml), followed by addition of 50% NH₂OH/H₂O (8 ml, 121 mmol). The mixture was stirred under atmospheric pressure at room temperature for 8 hours. The reaction was monitored by TLC (10% MeOH/AcOEt) and HPLC (0.1% AcOH-CH₃CN, 0-100%, 20 min, 254 nm). After confirming that methyl 4-(8-methoxy-8-oxooctanamido)benzoate 2 was completely consumed, hydroxylamine was quenched with acetic acid under ice cooling. The reaction mixture was dissolved in H₂O (10 ml) and then extracted three times with ethyl acetate (20 ml). The collected organic layer was dried over MgSO₄ and subjected to suction filtration to remove solids. The solvent was distilled off under reduced pressure to give 3 as a white solid.

Yield: 59.8 mg (28%).¹H NMR (CDCl₃, 400 MHz, δ): 10.3(brs, 1 H; NH), 7.96(d, J = 8.8 Hz, 2 H; CH), 7.79(d, J = 8.8 Hz, 2 H; CH), 3.88(s, 3 H; CH₃), 2.39-2.22(m, 4 H; CH₂), 1.72-1.61(m, 2 H; CH₂), 1.59-1.55(m, 2 H; CH₂), 1.40-1.33(m, 4 H; CH₂).

### 4-Aminobenzoic acid 4:

4-Aminobenzoic acid 4 was purchased from Tokyo Chemical Industry Co., Ltd., Japan.

### Synthesis of 4-(8-methoxy-8-oxooctanamido)benzoic acid 5:

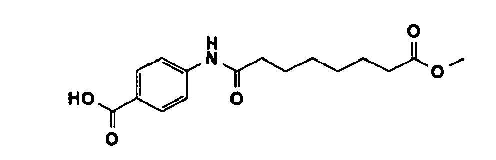

A 100 ml recovery flask was charged with 4-aminobenzoic acid 4 (2.1 g, 15.3 mmol). After fully degassing and drying, the flask was purged with Ar using a three-way stopcock. Then, dehydrated THF (20 ml) was added using a syringe to dissolve the reagent, followed by addition of DIEA (1.2 ml) as a base. After ice cooling, methyl 8-chloro-8-oxooctanoate (3.0 ml, 20.3 mmol) was added. The mixture was stirred for 8 hours while gradually warming to room temperature. The reaction was monitored by TLC (10% MeOH/AcOEt) and HPLC (0.1% AcOH-CH₃CN, 0-100%, 20 min, 254 nm). After confirming that 4-aminobenzoic acid 4 was completely consumed, water (2 ml) was added to quench the reaction. Subsequently, the solvent was distilled off under reduced pressure and the residue was dried, followed by purification on a silica gel column (eluent: AcOEt:MeOH = 9: 1). A fraction containing the desired product was collected, and the solvent was distilled off to dryness under reduced pressure to give 5 as a white solid.

Yield: 3.79 g (80%), ¹H NMR (DMSO-D₆, 400 MHz, δ): 10.2 (s, 1 H; NH), 7.85(d, J= 8.8 Hz, 2 H; CH), 7.69(d, J = 8.8 Hz, 2 H; CH), 3.60(s, 3 H; CH₃), 2.34-2.18(m, 4 H; CH₂), 1.57-1.50(m, 4 H; CH₂), 1.28-1.09(m, 4 H; CH₂). ESI-MS m/z calcd for C₁₆H₂₂NO₅₊; [M+H]⁺ 308.1, found 308.2.

### Synthesis of H₂NββImPyPyγImImPyβDp polyamide 6:

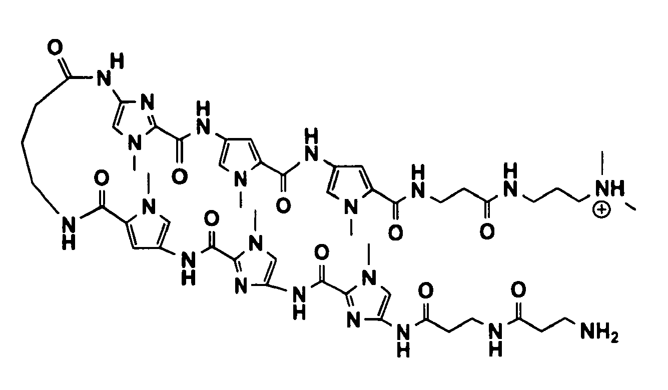

A Libra tube was charged with β-CLEAR resin (185 mg, 0.094 mmol), and 9 Eppendorf tubes were each charged with 1-[bis(dimethylamino)methylene]-5-chloro-1H-benzotriazolium-3-oxide hexafluorophosphate (HCTU; 160 mg, 0.39 mmol). Among these 9 tubes, 3 tubes were further charged with 4-(Fmoc-amino)-1-metyl-1H-pyrrole-2-carboxylic acid (145 mg, 0.4 mmol), another 3 tubes were further charged with 4-(Fmoc-amino)-1-metyl-1H-imidazole-2-carboxylic acid (145 mg, 0.4 mmol), 2 tubes were further charged with N-β-Fmoc-β-alanine (130 mg, 0.42 mmol), and the remaining one tube was further charged with N-γ-Fmoc-γ-aminobutyric acid (150 mg, 0.46 mmol). All of the samples were dried under reduced pressure. After fully drying, DMF (2 ml) was added to the Libra tube to swell the resin and then immediately distilled off. A 20% piperidine/DMF solution (3 ml) was added for deprotection to remove Fmoc groups. Removal of Fmoc was confirmed by measuring UV-Vis absorption. After deprotection, the solid phase was washed with CH₂Cl₂ (5 ml), methanol (5 ml) and DMF (5 ml). Then, the first mixture of reaction reagent and HCTU prepared above was added to the Libra tube, to which DMF (5 ml) and DIEA (70 µl) as a base were then added and dissolved. The mixture was reacted under stirring at room temperature for 1 hour. After 1 hour, acetic anhydride (0.2 ml) was added and reacted for 10 minutes to protect unreacted amino terminals with acetyl groups to thereby prevent further elongation reaction. Subsequently, the solvent was distilled off and the solid phase was washed with CH₂Cl₂ (5 ml), methanol (5 ml) and DMF (5 ml), followed by Fmoc deprotection with a 20% piperidine/DMF solution (3 ml). The same treatment was then repeated until the intended sequence was obtained. The last reagent was used in the reaction and deprotected to remove Fmoc. After drying, the entire solid phase was transferred to a 10 ml recovery flask, to which N,N-dimethyl-1,3-propanediamine (1 ml) was then added and stirred at 55°C for 8 hours, followed by excision of the resulting polyamide from the solid phase. The generation of desired polyamide 6 was confirmed by HPLC (0.1% AcOH-CH₃CN, 0-100%, 20 min, 254 nm) and ESI-MS. N,N-Dimethyl-1,3-propanediamine was distilled off and the polyamide was washed with Et₂O to give 6 as a yellow brown solid, which was obtained in a crude yield of 43.9 mg (41%).

ESI-MS m/z calcd for C₅₁H₇₀N₂₁O₁₀⁺ [M+H]⁺ 1136.5, found 1137.0.

### Synthesis of H₂NββImPyPyβImPyγImPyβImImPyββDp polyamide 7:

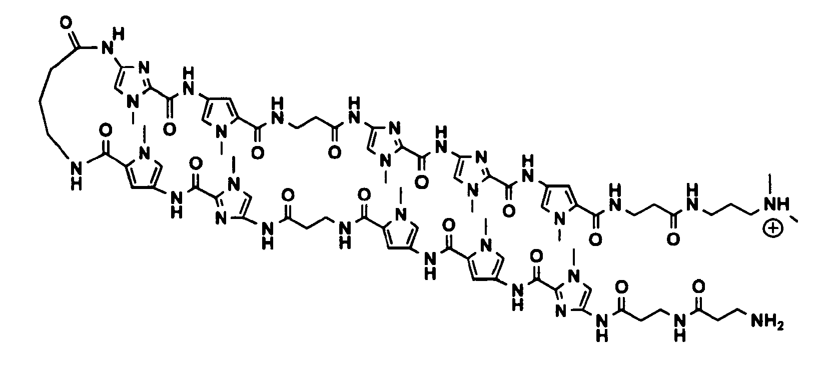

A Libra tube was charged with β-CLEAR resin (241.4 mg, 0.122 mmol), and 15 Eppendorf tubes were each charged with 1-[bis(dimethylamino)methylene]-5-chloro-1H-benzotriazolium-3-oxide hexafluorophosphate (160 mg, 0.39 mmol). Among these 15 tubes, 5 tubes were further charged with 4-(Fmoc-amino)-1-metyl-1H-pyrrole-2-carboxylic acid (145 mg, 0.4 mmol), another 5 tubes were further charged with 4-(Fmoc-amino)-1-metyl-1H-imidazole-2-carboxylic acid (145 mg, 0.4 mmol), 4 tubes were further charged with N-β-Fmoc-β-alanine (130 mg, 0.42 mmol), and the remaining one tube was further charged with N-γ-Fmoc-γ-aminobutyric acid (150 mg, 0.46 mmol). All of the samples were dried under reduced pressure. After fully drying, DMF (2 ml) was added to the Libra tube to swell the resin and then immediately distilled off. A 20% piperidine/DMF solution (3 ml) was added for deprotection to remove Fmoc protecting groups. Removal of Fmoc was confirmed by measuring UV-Vis absorption. After deprotection, the solid phase was washed with CH₂Cl₂ (5 ml), methanol (5 ml) and DMF (5 ml). Then, the first mixture of reaction reagent and HCTU prepared above was added to the Libra tube, to which DMF (5 ml) and DIEA (70 µl) as a base were then added and dissolved. The mixture was reacted under stirring at room temperature for 1 hour. After 1 hour, acetic anhydride (0.2 ml) was added and reacted for 10 minutes to protect unreacted amino terminals with acetyl groups to thereby prevent further elongation reaction. Subsequently, the solvent was distilled off and the solid phase was washed with CH₂Cl₂ (5 ml), methanol (5 ml) and DMF (5 ml), followed by Fmoc deprotection with a 20% piperidine/DMF solution (3 ml). The same treatment was then repeated until the intended sequence was obtained. The last reagent was used in the reaction and deprotected to remove Fmoc. After drying, the entire solid phase was transferred to a 10 ml recovery flask, to which N,N-dimethyl-1,3-propanediamine (1 ml) was then added and stirred at 55°C for 8 hours, followed by excision of the resulting polyamide from the solid phase. The generation of desired polyamide 7 was confirmed by HPLC (0.1% AcOH-CH₃CN, 0-100%, 20 min, 254 nm) and ESI-MS. N,N-Dimethyl-1,3-propanediamine was distilled off and the polyamide was washed with Et₂O to give 7 as a yellow brown solid, which was obtained in a crude yield of 50.4 mg (25%).

ESI-MS m/z calcd for C₇₉H₁₀₂N₃₃O₁₆⁺ [M+H]⁺ 1768.9, found 1769.2.

### Synthesis of H₂N(PEG)ββImPyPyβImPyγImPyβImImPyββDp polyamide 8:

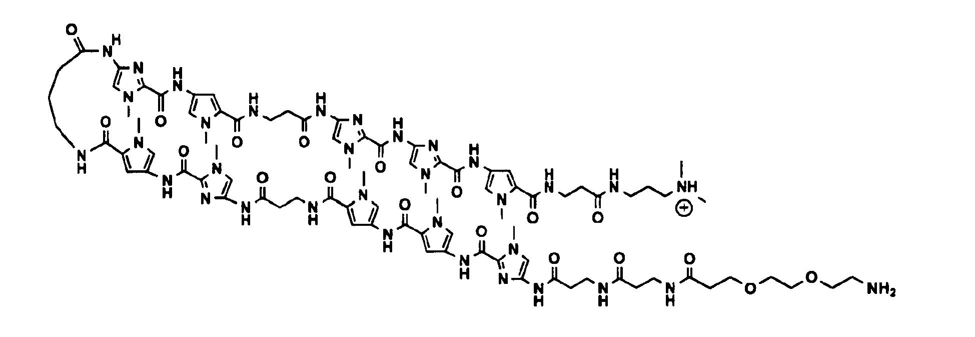

A Libra tube was charged with β-CLEAR resin (218.2 mg, 0.110 mmol), and 16 Eppendorf tubes were each charged with 1-[bis(dimethylamino)methylene]-5-chloro-1H-benzotriazolium-3-oxide hexafluorophosphate (160 mg, 0.39 mmol). Among these 16 tubes, 5 tubes were further charged with 4-(Fmoc-amino)-1-metyl-1H-pyrrole-2-carboxylic acid (145 mg, 0.4 mmol), another 5 tubes were further charged with 4-(Fmoc-amino)-1-metyl-1H-imidazole-2-carboxylic acid (145 mg, 0.4 mmol), 4 tubes were further charged with N-β-Fmoc-β-alanine (130 mg, 0.42 mmol), one tube was further charged with N-γ-Fmoc-γ-aminobutyric acid (150 mg, 0.46 mmol), and the remaining one tube was further charged with Fmoc-8-amino-3,6-dioxaoctanoic acid (150 mg, 0.39 mmol). All of the samples were dried under reduced pressure. After fully drying, DMF (2 ml) was added to the Libra tube to swell the resin and then immediately distilled off. A 20% piperidine/DMF solution (3 ml) was added for deprotection to remove Fmoc protecting groups. Removal of Fmoc was confirmed by measuring UV-Vis absorption. After deprotection, the solid phase was washed with CH₂Cl₂ (5 ml), methanol (5 ml) and DMF (5 ml). Then, the first mixture of reaction reagent and HCTU prepared above was added to the Libra tube, to which DMF (5 ml) and DIEA (70 µl) as a base were then added and dissolved. The mixture was reacted under stirring at room temperature for 1 hour. After 1 hour, acetic anhydride (0.2 ml) was added and reacted for 10 minutes to protect unreacted amino terminals with acetyl groups to thereby prevent further elongation reaction. Subsequently, the solvent was distilled off and the solid phase was washed with CH₂Cl₂ (5 ml), methanol (5 ml) and DMF (5 ml), followed by Fmoc deprotection with a 20% piperidine/DMF solution (3 ml). The same treatment was then repeated until the intended sequence was obtained. The last reagent was used in the reaction and deprotected to remove Fmoc. After drying, the entire solid phase was transferred to a 10 ml recovery flask, to which N,N-dimethyl-1,3-propanediamine (1 ml) was then added and stirred at 55°C for 8 hours, followed by excision of the resulting polyamide from the solid phase. The generation of desired polyamide 8 was confirmed by HPLC (0.1% AcOH-CH₃CN, 0-100%, 20 min, 254 nm) and ESI-MS. N,N-Dimethyl-1,3-propanediamine was distilled off and the polyamide was washed with Et₂O to give 8 as a yellow brown solid, which was obtained in a crude yield of 53.0 g (22%).

ESI-MS m/z calcd for C₈₅H₁₁₃N₃₄O₁₉⁺ [M+H]⁺ 1913.9, found 1914.2.

### Synthesis of 4-(8-methoxy-8-oxooctanamido)benzoyl-ββImPyPyγImlmPyβDp 9:

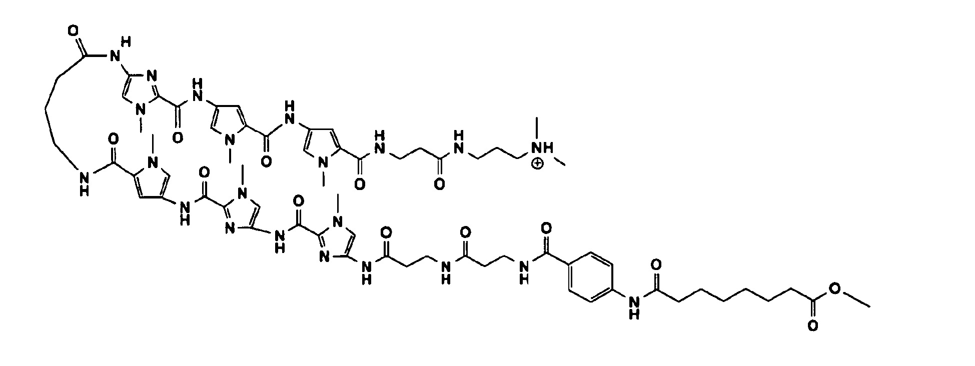

A 10 ml recovery flask was charged with 4-(8-methoxy-8-oxooctanamido)benzoic acid 5 (68.3 mg, 0.22 mmol), H₂N-ββImPyPyγImImPyβDp 6 (57.2 mg, 0.05 mmol) and FDPP (67.5 mg, 0.21 mmol). After degassing and drying, the flask was purged with Ar using a three-way stopcock. Then, dehydrated DMF (4 ml) and DIEA (0.2 ml) as a base were added, and the mixture was stirred at room temperature for 6 hours. The reaction was monitored by HPLC (0.1% AcOH-CH₃CN, 0-100%, 20 min, 254 nm). After confirming that H₂N-ββImPyPyγImImPyβDp 6 was completely consumed, the solvent was distilled off under reduced pressure and Et₂O (3 ml) was then added to dissolve soluble components, followed by filtration to collect only insoluble components. The collected insoluble components were dissolved in DMF (1 ml) and purified by HPLC on a Chemcobond 5-ODS-H column (0.1% AcOH/CH₃CN 0-100%, 20 min, 360 nm) to give 9 as a white solid.

Yield: 14.6 mg (19%). ¹H NMR (DMSO-D₆, 400 MHz, δ): 10.4(s, 1 H; NH), 10.3(s, 2 H; NH), 10.1(s, 1 H; NH), 9.97(s, 1 H; NH), 9.92(s, 1 H; NH), 9.33(s, 1 H; NH), 8.42-8.22(m, 1 H; CH), 8.16(d, J = 8.0 Hz, 1 H; CH), 8.10-7.99(m, 2 H; CH), 7.94(s, 2 H; CH), 7.76-7.74(m, 2 H; CH), 7.64(d, J = 8.8 Hz, 1 H; CH), 7.56(s, 1 H; CH), 7.50(s, 1 H, CH), 7.45(s, 1 H; CH), 7.27(d, J = 8.8 Hz, 2 H; CH), 7.20(s, 1 H; CH), 7.18(s, 1 H;CH). 7.13(s, 1 H: CH), 7.00(s, 1 H; CH), 6.85(s, 1 H, CH), 4.00-3.84(m, 8 H; CH₂), 3.80-3.56(m, 8 H; CH₂), 3.49(s, 3 H; CH₃), 3,20-3,16(m, 4 H; CH₂), 3.10-3.00(m, 4 H; CH₂), 2.87(d, J= 4.8 Hz, 6 H; CH₂), 2.71(d, J = 4.8 Hz, 6 H; CH₂), 2.34-2.18 (m, 4 H; CH₂), 1.78-1.66(m, 2 H; CH₂), 1.57-1.50(m, 4 H; CH₂), 1.28-1.09(m, 4 H; CH₂). ESI-MS m/z calcd for C₆₇H₈₉N₂₂O₁₄⁺ [M+H]⁺ 1425.7, found 1426.0.

### Synthesis of 4-(8-(benzyloxyamino)-8-oxooctanamido)phenylamido-ββPyPyImγImImPyβDp 14:

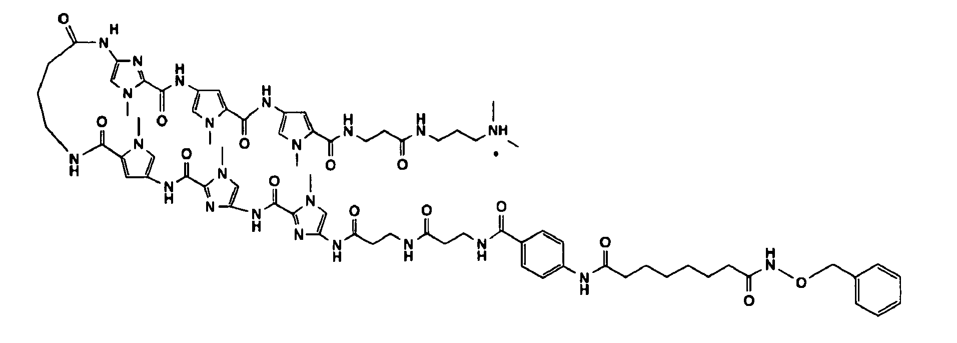

To a 10 ml recovery flask, 4-(8-methoxy-8-oxooctanamido)benzoyl-ββImPyPyγImImPyβDp 9 (3.4 mg, 2.4 µmol) and LiOH·H₂O (3.5 mg, 0.084 mol) were added and dissolved in a mixed solvent of DMF (300 µl) and H₂O (50 ml), followed by stirring at room temperature for 12 hours. The reaction was monitored by HPLC (0.1% AcOH-CH₃CN, 0-100%, 20 min, 254 nm). After confirming that 4-(8-methoxy-8-oxooctanamido)benzoyl-ββImPyPyγImImPyβDp 9 was hydrolyzed to give polyamide 13 by ESI-MS (positive) analysis, O-benzylhydroxylamine hydrochloride (11.2 mg, 0.070 mmol), FDPP (23.4 mg, 0.073 mmol), DMF (200 µl), H₂O (50 µl), and DIEA (100 µl) as a base were added and stirred at room temperature for 12 hours. The reaction was monitored by HPLC (0.1% AcOH-CH₃CN, 0-100%, 20 min, 254 nm). After confirming that polyamide 13 was completely consumed, the reaction was quenched with acetic acid under ice cooling and the solvent was then distilled off under reduced pressure. The resulting residue was dissolved in DMF (1 ml) and purified by HPLC on a Chemcobond 5-ODS-H column (0.1% AcOH/CH₃CN 0-100%, 20 min, 360 nm) to give 14 as a white solid.

Yield: 2.1 mg (56%), ¹H NMR (DMSO-D₆, 400 MHz, δ): 11.1(s, 1 H, NH), 11.0(s, 1 H, NH), 11.9(s, 1 H, NH), 10.4(s, 1 H; NH), 10.3(s, 2 H; NH), 10.1(s, 1 H; NH), 9.95(s, 1 H; NH), 9.92(s, 1 H; NH), 9.35(s, 1 H; NH), 8.42-8.30(m, 1 H; CH), 8.23(s, 1 H; CH), 8.1-8.02(m, 1 H; CH), 8.00-7.96(m, 2 H; CH), 7.94(s, 2 H; CH), 7.84(brs, 2 H; NH) 7.76(s, 1 H; CH), 7.76(m, 2 H; CH), 7.74(s, 1 H; CH), 7.70-7.60(m, 5H; CH), 7.55(s, 1 H; CH), 7.50(s, 1 H; CH), 7.44(s, 1 H; CH), 7.38-7.34 (m, 4 H; CH), 7.33-7.26(m, 8 H; CH), 7.20(s, 1 H; CH). 7.13(s, 1 H; CH), 7.17(s, 1 H, CH), 7.00(s, 1 H; CH), 6.86(s, 1 H, CH), 4.00-3.84(m, 8 H; CH₂), 3.80-3.56(m, 8 H; CH₂), 3.20-3.00(m, 12 H; CH₂), 2.91(s, 6 H; CH₂), 2.72(s, 6 H; CH₂ 2.34-2.18(m, 4 H; CH₂), 1.78-1.66(m, 2 H; CH₂), 1.57-1.50(m, 4 H; CH₂), 1.22-1.12(m, 4 H; CH₂). ESI-MS m/z calcd for C₇₃H₉₄N₂₃O₁₄⁺ [M+H]⁺ 1516.7, found 1517.0.

### Synthesis of SAHA-ββImPyPyγImImPyβDp 12 (SAHA+non-targeting PIP):

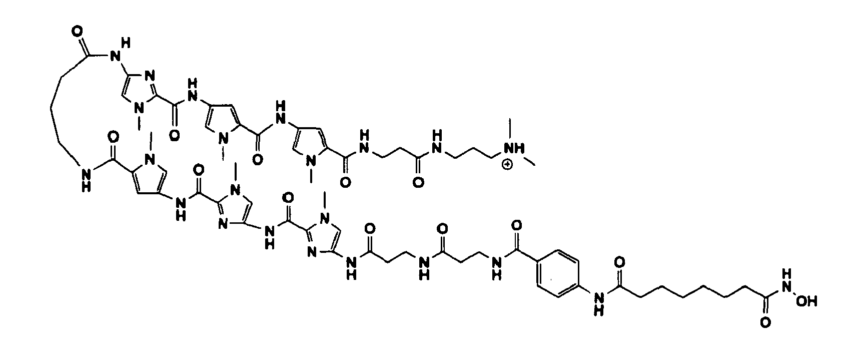

In a 10 ml recovery flask, 4-(8-methoxy-8-oxooctanamido)benzoyl-ββImPyPyγImImPyβDp 9 (14.8 mg, 10.0 µmol) was dissolved in DMF (3.0 ml) and mixed with 50% NH₂OH/H₂O (1.5 ml, 22.6 mmol), followed by stirring at room temperature for 10 hours. The reaction was monitored by HPLC (0.1% AcOH-CH₃CN, 0-100%, 20 min, 254 nm) to obtain a chart which was significantly broadened as compared to that of starting material 9. After confirming that 4-(8-methoxy-8-oxooctanamido)benzoyl-ββImPyPyγImImPyβDp 9 was completely consumed, the reaction was quenched with acetic acid under ice cooling and the solvent was then distilled off under reduced pressure. The resulting residue was dissolved in DMF (1 ml) and purified by HPLC on a Chemcobond 5-ODS-H column (50 mmol AF/CH₃CN 0-100%,20 min, 360 nm) to give 12 as a white solid.

Yield: 3.8 mg (26%). ¹H NMR (DMSO-D₆, 400 MHz, δ): 10.4(s, 1 H; NH), 10.3(s, 2 H; NH), 10.1(s, 1 H;NH), 10.2(s, 1 H; NH), 10.1(s, 1 H; NH), 9.93(s, 1 H; NH), 9.92(s, 1 H,NH), 9.88(s, 1 H; NH), 9.33(s, 1 H; NH), 8.42-8.22(m, 4 H, CH), 8.10-7.94(m, 4 H, CH), 7.86-7.79(m, 2 H; CH), 7.76-7.73(m, 2 H; CH), 7.64 (d, J= 8.8 Hz, 1 H; CH), 7.56 (s, 1H; CH), 7.50(s, 1 H, CH), 7.45(s, 1 H; CH), 7.27(d, J = 8.8 Hz, 2 H; CH), 7.20(s, 1 H; CH), 7.17(s, 1 H; CH). 7.11 (s, 1 H: CH), 7.00(s, 1 H; CH), 6.80(s, 1 H, CH), 4.00-3.84(m, 8 H; CH₂), 3.80-3.56(m, 8 H; CH₂), 3.49(s, 3 H; CH₃), 3.20-3.16(m, 4 H; CH₂), 3.10-3.00(m, 4 H; CH₂), 2.87(d, J= 4.8 Hz, 6 H, CH₂), 2.71(d, J = 4.8 Hz, 6 H; CH₂),2.34-2.18 (m, 4 H; CH₂), 1.78-1.66(m, 2 H; CH₂), 1.57-1.50(m, 4 H; CH₂), 1.28-1.09(m, 4 H; CH₂). ESI-MS m/z calcd for C₆₇H₈₉N₂₂O₁₄⁺ [M+H]⁺ 1426.7, found 1427.0

### Synthesis of SAHA-ββImPyPyγImImPyβDp12 (SAHA+non-targeting PIP) from 4-(8-(benzyloxyamino)-8-oxooctanamido)phenylamido-ββPyPyImγImImPyβDp 14:

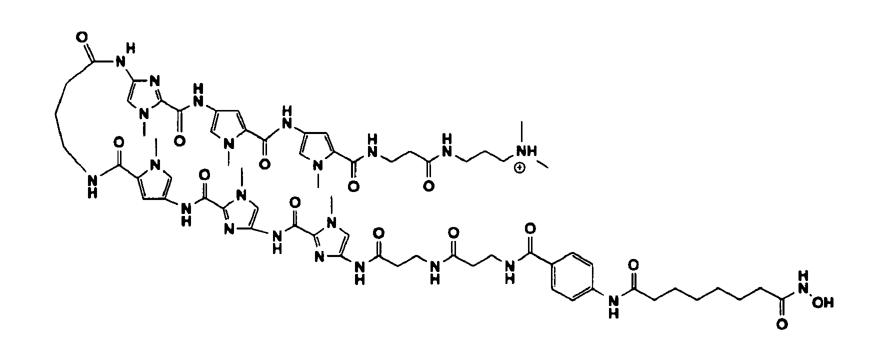

A 10 ml recovery flask was charged with 4-(8-(benzyloxyamino)-8-oxooctanamido)phenylamido-ββPyPyImγImImPyβDp 14 (3.2 mg, 2.1 µmol) and 10% Pd/C (1.1 mg). After fully degassing and drying, the flask was purged with H₂ gas using a three-way stopcock. Dehydrated methanol (0.5 ml) was added and the mixture was stirred at room temperature under balloon pressure for 16 hours. The reaction was monitored by HPLC (0.1% AcOH-CH₃CN, 0-100%, 20 min, 254 nm) to obtain a significantly broadened chart for the mixture. Solids were removed by suction filtration and the solvent was distilled off to give a white solid of the mixture (1.4 mg). A trace amount of SAHA-ββImPyPyγImimPyβDp 12 was confirmed by HPLC analysis (0.1% AcOH-CH₃CN, 0-100%, 20 min, 254 nm) and ESI-MS, but it was not a main product. ESI-MS m/z calcd for C₆₇H₈₉N₂₂O₁₄⁺ [M+H]⁺ 1426.7, found 1427.0

### Synthesis of 4-(8-methoxy-8-oxooctanamido)benzoyl-ββImPyPyβImPyγImPyβImIm PyβDp 10 (esterified SAHA+p16 PIP):

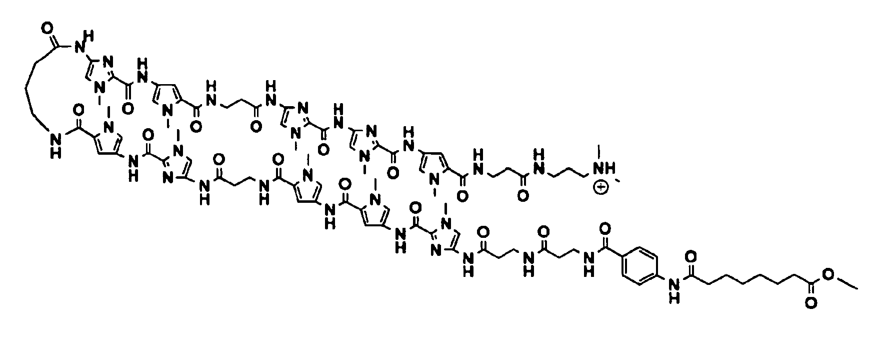

A 10 ml recovery flask was charged with 4-(8-methoxy-8-oxooctanamido)benzoic acid 5 (16.9 mg, 0.054 mmol), H₂N-ββImPyPyβImPyγImPyβImImPyβDp 7 (20.2 mg 11.0 µmol) and FDPP (68.4 mg, 0.22 mmol). After degassing and drying, the flask was purged with Ar using a three-way stopcock. Then, dehydrated DMF (1.5 ml) and DIEA (0.15 ml) as a base were added, and the mixture was stirred at room temperature for 6 hours. The reaction was monitored by HPLC (0.1% AcOH-CH₃CN, 0-100%, 20 min, 254 nm). After confirming that H₂N-ββImPyPyβImPyγImPyβImImPyβDp 7 was completely consumed, the solvent was distilled off under reduced pressure. Et₂O (3 ml) was added to dissolve and remove soluble components, and only insoluble components were collected. The collected insoluble components were dissolved in DMF (1 ml) and purified by HPLC on a Chemcobond 5-ODS-H column (0.1% AcOH/CH₃CN 0-100%, 20 min, 360 nm) to give 10 as a white solid.

Yield: 8.0 mg (38%). ESI-MS m/z calcd for C₉₅H₁₂₁N₃₄O₂₀⁺, [M+H]⁺ 2058.9, found 2059.2.

### Synthesis of SAHA-ββImPyPyβImPyγImPyβImImPyβDp 15 (SAHA+p16 PIP):

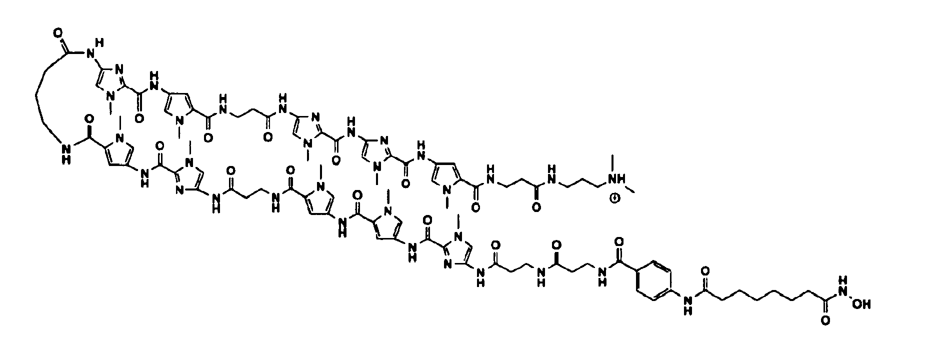

In a 10 ml recovery flask, 4-(8-methoxy-8-oxooctanamido)benzoyl-ββImPyPyβImPyγImPyβImImPyβDp 10 (8.0 mg, 4.0 µmol) was dissolved in DMF (2.0 ml) and mixed with 50% NH₂OH/H₂O (1.0 ml, 15.1 mmol), followed by stirring at room temperature for 10 hours. The reaction was monitored by HPLC (0.1% AcOH/CH₃CN 0-100%, 0-100%, 20 min, 254 nm). After confirming that 4-(8-methoxy-8-oxooctanamido)benzoyl-ββImPyPyβImPyγImPyβImImPyβDp 10 was completely consumed, the reaction was quenched with acetic acid under ice cooling and the solvent was then distilled off under reduced pressure. The resulting residue was dissolved in DMF (1 ml) and purified by HPLC on a Chemcobond 5-ODS-H column (50 mmol AF-CH₃CN, 20 min, 360 nm) to give 15 as a white solid.

Yield: 5.0 mg (61%). ¹H NMR (DMSO-D₆, 400 MHz, δ): ESI-MS m/z calcd for C₉₄H₁₂₀N₃₅O₂₀⁺. [M+H]⁺ 2059.9, found 2060.2.

### Synthesis of 4-(8-methoxy-8-oxooctanamido)benzoyl-(PEG) ββImPyPyβImPyγImPyβImIm PyβDp 11:

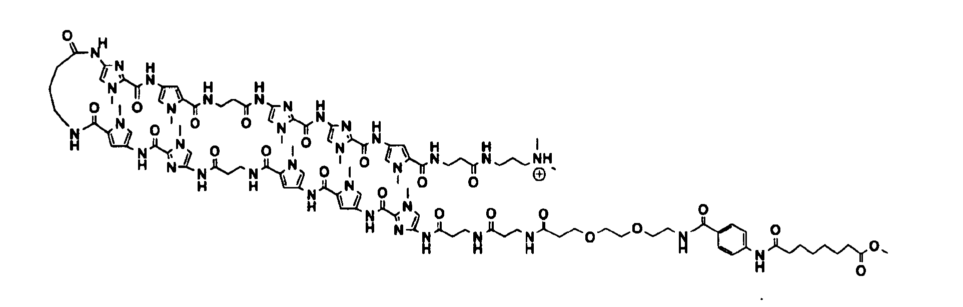

A 10 ml recovery flask was charged with 4-(8-methoxy-8-oxooctanamido)benzoic acid 5 (67.1 mg, 0.22 mmol), H₂N-ββImPyPyβImPyγImPyβImImPyβDp 8 (47.8 mg 25.0 µmol) and FDPP (50.0 mg, 0.16 mmol). After degassing and drying, the flask was purged with Ar using a three-way stopcock. Then, dehydrated DMF (4.0 ml) and DIEA (0.20 ml) as a base were added, and the mixture was stirred at room temperature for 12 hours. The reaction was monitored by HPLC (0.1% AcOH-CH₃CN, 0-100%, 20 min, 254 nm). After confirming that H₂N-(PEG) ββImPyPyβImPyγImPyβImImPyβDp 8 was completely consumed, the solvent was distilled off under reduced pressure. Et₂O (3 ml) was added to dissolve and remove soluble components, and only insoluble components were collected. The collected insoluble components were dissolved in DMF (1 ml) and purified by HPLC on a Chemcobond 5-ODS-H column (0.1% AcOH/CH₃CN 0-100%, 20 min, 360 nm) to give 11 as a white solid.

Yield: 6.8 mg (12%). ESI-MS m/z calcd for C₁₀₁H₁₃₁N₃₅O₂₄⁺, [M+H]⁺ 2203.0, found 2204.2.

### Synthesis of SAHA-(PEG) ββImPyPyβImPyγImPyβImImPyβDp 16 (SAHA-ether linker+p16 PIP):

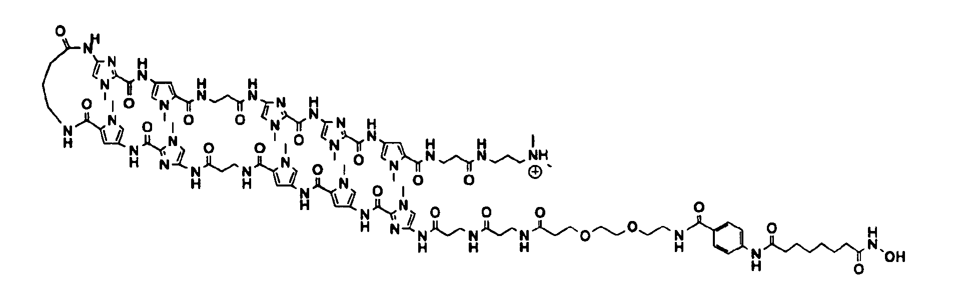

In a 10 ml recovery flask, 4-(8-methoxy-8-oxooctanamido)benzoyl-(PEG) ββImPyPyβImPyγImPyβImImPyβDp 11 (6.8 mg, 3.0 µmol) was dissolved in DMF (3.0 ml) and mixed with 50% NH₂OH/H₂ (3.0 ml 45.3 mmol), followed by stirring at room temperature for 10 hours. The reaction was monitored by HPLC (0.1% AcOH-CH₃CN, 0-100%, 20 min, 254 nm). After confirming that 4-(8-methoxy-8-oxooctanamido)benzoyl-(PEG) ββImPyPyβImPyγImPyβImImPyβDp 11 was completely consumed, the reaction was quenched with acetic acid under ice cooling and the solvent was then distilled off under reduced pressure. The resulting residue was dissolved in DMF (1 ml) and purified by HPLC on a Chemcobond 5-ODS-H column (50 mmol AF-CH₃CN 0-100%,20 min, 360 nm) to give 16 as a white solid.

Yield: 0.8 mg (7.6%). ESI-MS m/z calcd for C₁₀₀H₁₃₀₁N₃₆O₂₃⁺. [M+H]⁺ 2204.0, found 2205.4.

### 3. HDAC activity assay:

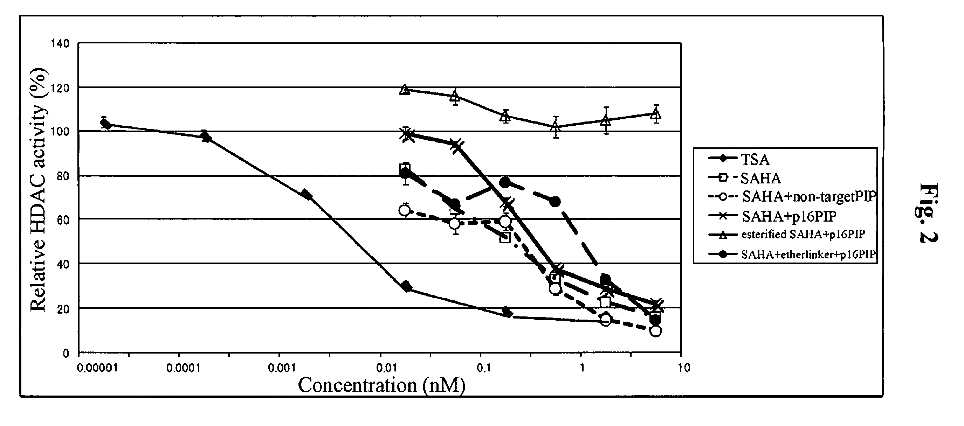

To verify whether the activity of histone deacetylase (HDAC) was inhibited by compounds composed of the synthesized SAHA derivatives and polyamides attached to each other, an HDAC Fluorimetric Assay/Drug Discovery Kit (BIOMOL international) was used in the experiment. Although details followed the instructions of the kit manufacturer, an HDAC-containing HeLa cell (human uterine cervical cancer cell line) nuclear fraction was added to an acetylation substrate and incubated at 37°C for 1 hour in the presence of various concentrations of the synthesized compounds or trichostatin A (hereinafter referred to as TSA) used as a positive control, followed by addition of the developer included in the kit to stop the HDAC-catalyzed reaction. Then, the samples were transferred to a 96-well assay plate and measured with ARVO EX (Perkin Elmer) for emission at 460 nm from the reaction product upon excitation at 360 nm. The results obtained are shown in Figure 2.

Figure 2 indicated that all the synthesized SAHA derivatives had anti-HDAC activity, and this activity was not affected by polyamide attachment. Moreover, it was also indicated that the conjugate between esterified SAHA and p16 PIP, which was prepared for use as a negative control, did not inhibit HDAC activity.

In view of the foregoing, SAHA+p16 PIP, SAHA+non-targeting PIP and SAHA+ether linker+p16 PIP were confirmed to have anti-HDAC activity. In contrast, esterified SAHA+p16 PIP had no HDAC activity.

### 4. Cell proliferation assay (I):

Next, the synthesized compounds were verified by WST-8 assay for their ability to suppress cancer cell proliferation.

Cell lines of HeLa (human uterine cervical cancer cell line), LOVO, RKO and SW480 (each of which was a human large bowel cancer cell line) were each cultured at 37°C under 5% carbon dioxide conditions using Dulbecco's modified Eagle's medium (DMEM, Nacalai Tesque, Inc., Japan) supplemented with 10% fetal bovine serum (FBS: JRH bioscience, USA), antibiotics (50 U/ml penicillin and 50 µg/ml streptomycin) and 1 mM sodium pyruvate. Single-layered cells grown to 80-90% confluency on 10 cm cell culture dishes were washed with D-PBS(-) (Nacalai Tesque, Inc., Japan) and then released from the dishes by being treated with a phenol red-containing 2.5 g/L-Trypsine/1 mmol/L EDTA solution (Nacalai Tesque, Inc., Japan). The released cells were adjusted and maintained in fresh medium.

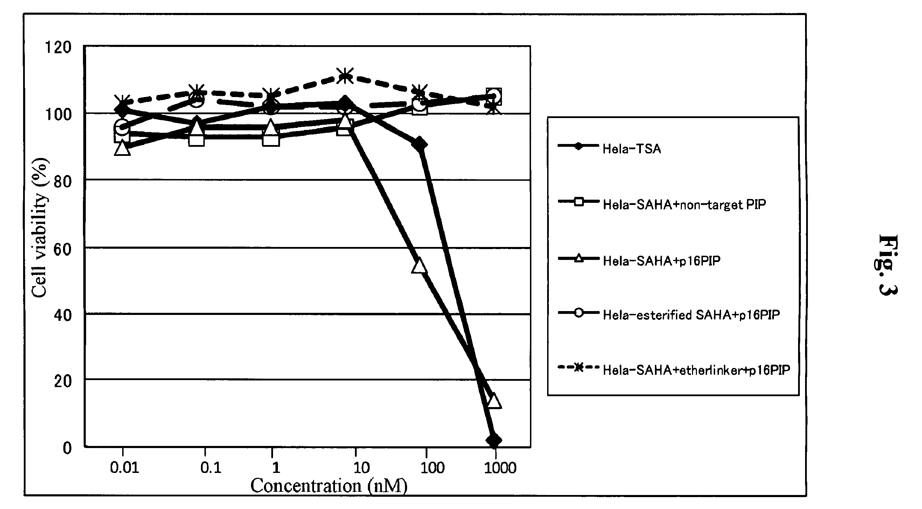

Each cell line seeded at 3 × 10³ cells/well in a MICROTEST tissue culture plate (96 well, FALCON) was cultured at 37°C under 5% carbon dioxide conditions for 24 hours, and then cultured in the presence of various concentrations of PI polyamide for an additional 72 hours. After completion of the culture, 10 µl of viable cell counting reagent SF (Nacalai Tesque, Inc., Japan) was added to each well and the absorbance at 450 nm was measured by ARVO EX (PerkinElmer). During the period from reagent addition until assay, incubation was continued at 37°C in the presence of 5% CO₂ until the absorbance reached about 1.2 to 2.0 in the control sample where only 50% DMSO was added to the cells. The results obtained are shown in Figure 3.

Figure 3 indicated that p16 PIP attached to the SAHA derivative showed proliferation inhibition against HeLa cells, as in the case of TSA used as a positive control. On the other hand, when HeLa cells were treated with non-targeting PIP or esterified SAHA+p16 PIP, their proliferation was not suppressed. This indicates that proliferation is not suppressed by p16 PIP alone, that proliferation is also not suppressed when SAHA is guided to DNA sequences by non-targeting PIP designed not to recognize the gene regulatory region of p16, and that cell proliferation is suppressed by cooperation between SAHA derivative and p16 PIP. Further, TSA shows high activity to HDAC even at low concentrations, and the same ability to suppress cell proliferation as observed in TSA was also observed in the conjugate between SAHA derivative and p16 PIP at substantially the same or higher concentration, thus suggesting a possibility that the concentration of HDAC inhibitors can be reduced when the target site on the genome is limited.

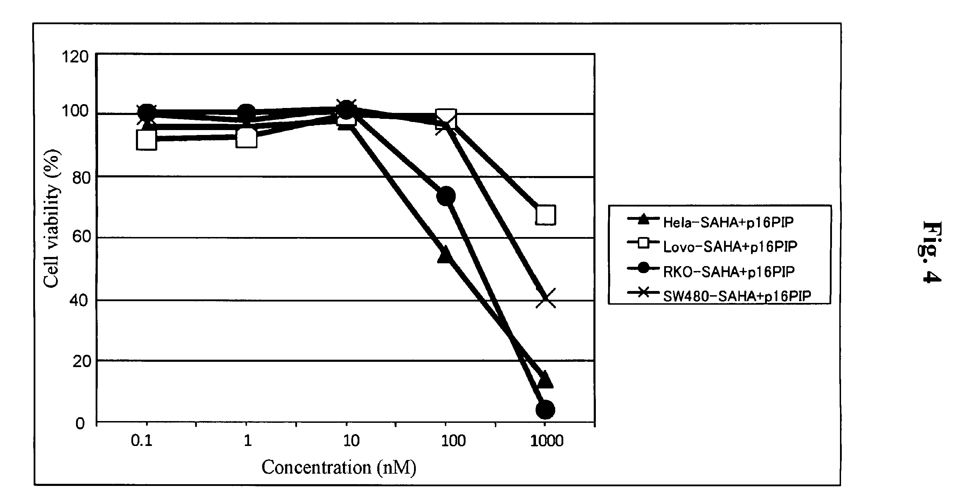

Figure 4 shows the results of cell proliferation assay in various cancer cells. p16 PIP attached to the SAHA derivative was found to inhibit proliferation of all the 4 cancer cell lines HeLa, LOVO, RKO and SW480 used in the experiment. Its ability to suppress proliferation was high in HeLa and RKO, but the proliferation of LOVO or RKO was not suppressed at 100 nM. The ability was lowest in RKO.

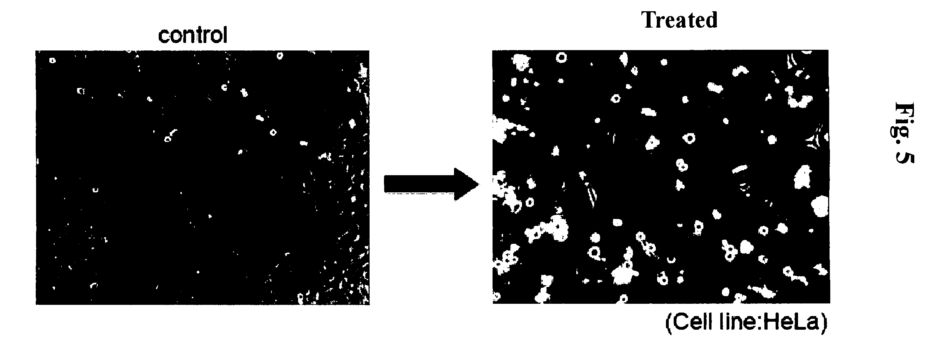

Moreover, when the cultured HeLa cell line was observed under an inverted optical microscope of 20 magnification, there was a significant change in its cell morphology, as shown in Figure 5. Namely, the cell density was reduced and many flattened cells were observed.

In view of the foregoing, SAHA+p16 PIP was confirmed to have a high antitumor effect.

### 5. Real-time RT-PCR:

To verify the effect on p16 gene expression, real-time RT-PCR was performed. HeLa cells seeded at 1.5 × 10⁴ cells per well in 24-well plates were cultured for 48 hours in the presence of various concentrations of the polyamides, and RNA was then collected by ISOGEN (Nippon Gene Co., Ltd., Tokyo, Japan). The procedures used followed the manufacturer's instructions. The isolated total RNA was measured for its concentration with Nanodrop (NanoDrop, USA) and used for cDNA synthesis with a Primescript RT reagent kit (TAKARA BIO, Shiga, Japan). The prepared cDNA was confirmed for the expression level of p 16 RNA in a Thermal Cycler Dice Real-time System TP-800 (TAKARA BIO, Shiga, Japan) with SYBR premix EX Taq (TAKARA BIO, Shiga, Japan). In this case, the following primers were used in PCR reaction for p16 amplification.
Forward:
5'-GGCACCAGAGGCAGTAACCA-3' (SEQ ID NO: 2),
Reverse:
5'-GGACCTTCGGTGACTGATGATCTAA-3' (SEQ ID NO: 3).

Likewise, for amplification of human endogenous glyceraldehyde-3-phosphate dehydrogenase (GAPDH) used as an internal standard, the following primers were used in PCR reaction.
Forward:
5'-GCACCGTCAAGGCTGAGAAC-3' (SEQ ID NO: 4),
Reverse:
5'-TGGTGAAGACGCCAGTGGA-3' (SEQ ID NO: 5).

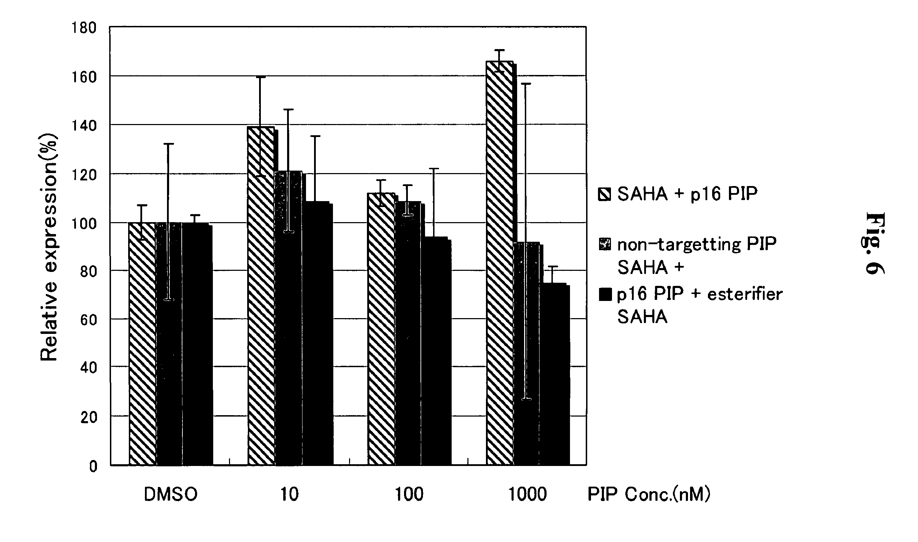

The results obtained are shown in Figure 6. Figure 6 indicated that SAHA+p16 PIP caused a significant increase in the expression level of p16 even at 10 nM concentration at the transcription level. In contrast, the other two drugs used as negative controls showed no statistical significance, thus indicating that it is necessary to use a conjugate between SAHA derivative and p16 PIP for enhanced expression of p16 mRNA.

### 6. Western blotting:

After being cultured for 48 hours in the presence of various concentrations of polyamide, HeLa cells were harvested and lysed again in an extraction buffer containing 20 mM Tris-HCl (pH 7.5), 150 mM NaCl, 1 mM EDTA, 1% Nonidet P-40 and Complete Protease Inhibitor Cocktail Tablets (Roche), and then centrifuged at 13,500 x g at 4°C for 15 minutes. The resulting supernatants were supplemented with a sample buffer solution (2ME+, x4) (Wako Pure Chemical Industries, Ltd., Japan) to give samples. These samples were developed on a 15% polyacrylamide gel, electrically transferred to a polyvinylidene difluoride membrane (Millipore Corporation, USA), and then blocked with 5% skimmed milk for the purpose of preventing non-specific binding. The primary antibodies used for target protein detection were purified anti-human p16INK4 monoclonal antibody (BD bioscience) for p16 and α-tubulin (TU-02) (SANTA CRUZ) for α-tubulin used as an internal standard. In both cases, the secondary antibody used was blotting grade affinity purified goat anti-mouse IgG (H+L) horseradish peroxidase conjugate (BIO RAD). After antibody labeling, the membrane was developed with Immobilon western (MILLIPORE) and then detected by Las-4000 mini (Fuji Photo Film Co., Ltd., Japan). The area and intensity of each band were determined by Multigauge (Fuji Photo Film Co., Ltd., Japan) and quantified in comparison with a 50% DMSO-treated control, which was set to 100%.

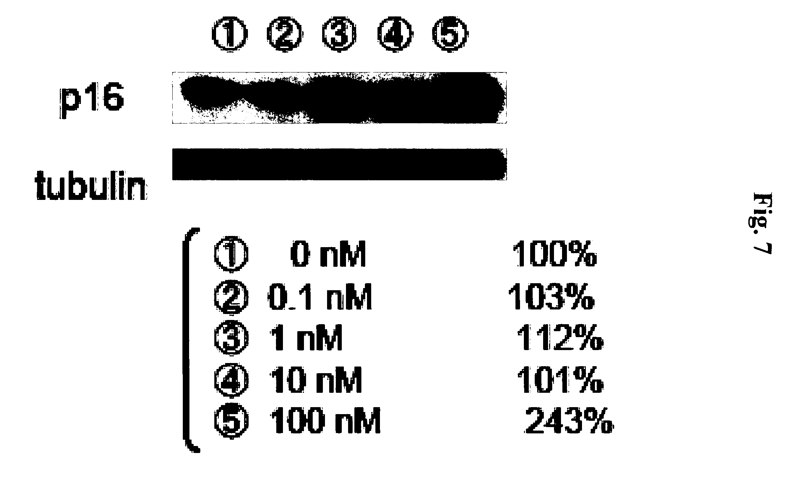

The results obtained are shown in Figure 7. Figure 7 indicated that SAHA+p16 PIP enhanced the expression of p16 not only at the mRNA level, but also at the protein level.

### 7. Genomic region-specific quantitative acetylation analysis by histone acetylation antibody precipitation:

In a 10% fetal bovine serum-containing DMEM medium prepared to contain the synthesized compound (SAHA+p16 PIP or SAHA+non-targeting PIP) or SAHA at a final concentration of 150 nM, HeLa cells were cultured at 37°C in the presence of 5% CO₂ for 7 days. After culture, 5 x 10⁶ to 1 x 10⁷ confluent cells were harvested in 100 mm diameter cell culture dishes (Corning 430167) and then subjected to chromatin immunoprecipitation assay with a MILIPORE 17-295 Chromatin Immunoprecipitation (ChIP) assay kit.

The detailed procedures used are as follows. To the harvested cells cultured for 7 days, formalin was added at a final concentration of 1% and incubated at 37°C for 10 minutes to fix the cells, whereby proteins and DNAs were crosslinked within the cells. Then, glycine was added at a final concentration of 0.25 M and the supernatant was removed, followed by washing the cell layer. Then, the cell layer was treated with 220 µl lysis buffer and the cell lysate was collected with a scraper. The resulting cell lysate was placed in water at 4°C and the cells in the cell lysate were crushed with a BIORUPTOR (SANYO) (power was set to H, and the cycle of 30 second sonication and 1 minute pause was repeated 10 times). Then, refrigerated centrifugation was performed at 15000 x g at 4°C for 10 minutes, and the resulting supernatant (200 µl) was supplemented with 1800 µl dilution buffer and 25 µl Protein A sepharose, followed by rotary mixing at 4°C for 30 minutes to remove non-specific binding. Then, centrifugation was performed at 1000 x g for 1 minute, and the resulting supernatant (400 µl) was supplemented with 2 µg of anti-histone H3 antibody (Abcam, Cat. No. ab1971, Lot 517069) or 2 µg of anti-histone H3K9 acetyl antibody (Abcam, Cat. No. ab10812, Lot 522309), followed by rotary mixing at 4°C for 12 hours. Then, 40 µl protein sepharose was added and rotary mixing was performed at 4°C for 1 hour, followed by centrifugation at 1000 x g for 1 minute to precipitate the sepharose. The precipitate was washed with the LOW buffer included in the kit and then centrifuged at 1000 x g for 1 minute to precipitate the sepharose. The precipitated sepharose was washed with High Buffer and then centrifuged at 1000 x g for 1 minute to precipitate the sepharose again. The precipitated sepharose was washed with LiCl₂ buffer and then centrifuged at 1000 x g for 1 minute to precipitate the sepharose again. The precipitated sepharose was washed with TE and then centrifuged at 1000 x g for 1 minute to precipitate the sepharose again. Then, the sepharose was suspended in 250 µl of a 0.1 M NaHCO₃-1% SDS solution and, after 15 minutes, centrifuged at 1000 x g for 1 minute to precipitate the sepharose again. After the supernatant was collected, the precipitated sepharose was suspended again in 250 µl of a 0.1 M NaHCO₃-1% SDS solution and, after 15 minutes, centrifuged at 1000 x g for 1 minute to precipitate the sepharose. The eluate in a total volume of 500 µl was collected and treated at 65°C for 4 hours in the presence of NaCl added at a final concentration of 200 mM. Then, the eluate was further treated at 45°C for 1 hour with protease K at a final concentration of 20 ng/µl. After phenol-chloroform extraction, DNA was purified by ethanol precipitation. Then, the purified DNA was used to amplify a target genomic region by PCR. PCR was accomplished in the same manner as described above in the section "5. Real-time RT-PCR." More specifically, the purified DNA was quantitatively analyzed with SYBR premix EX Taq (TAKARA BIO, Shiga, Japan) in a Thermal Cycler Dice Real-time System TP-800 (TAKARA BIO, Shiga, Japan). In this case, the following primer sets were used as primers for confirming chromatin immunoprecipitation of the p 16 or LARP 1 gene.

It should be noted that the reason why the analysis was performed not only on the p16 gene but also on the LARP1 gene is because the LARP1 gene region was found to include a sequence similar to the sequence of the regulatory region in the p16 gene recognized by SAHA+p16 PIP.
p16 forward: 5'-TTGCCAACGCTGGCTCTGG-3' (SEQ ID NO: 8)
p 16 reverse: 5'-CAAATCCTCTGGAGGGACCGC-3' (SEQ ID NO: 9)
LARP1 forward: 5'-TACCAACCACTGTCCCAGAG-3'(SEQ ID NO: 10)
LARP 1 reverse: 5'-TGGGTTTGAACTGTGTCTTG-3' (SEQ ID NO: 11)

Likewise, for amplification of the human endogenous glyceraldehyde-3-phosphate dehydrogenase (GAPDH) gene region used as an internal standard, the following primers were used in PCR reaction.
Forward:
5'-TACTAGCGGTTTTACGGGCG-3' (SEQ ID NO: 12)
Reverse:
5'-TCGAACAGGAGGAGCAGAGAGCGA-3' (SEQ ID NO: 13)

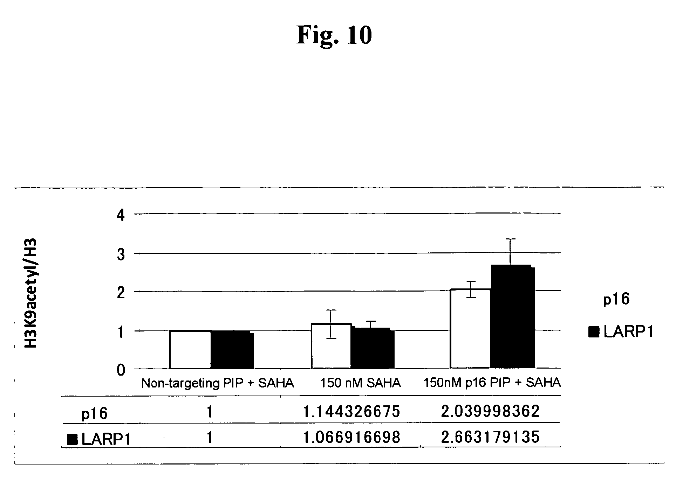

The results obtained are shown in Figure 10. As shown in Figure 10, SAHA+p16 PIP enhanced acetylation of histone H3 lysine 9 in the p16 genomic region, and showed about 2-fold significant enhancement when compared to SAHA alone or the conjugate between polyamide not recognizing the p16 region and SAHA (SAHA+non-targeting PIP). Moreover, in the LARP1 gene including a sequence recognized by SAHA+p16 PIP, which is similar to the sequence of the regulatory region in the p16 gene, SAHA+p16 PIP also enhanced acetylation of histone H3 lysine 9 in the genomic region of the LARP1 gene, and showed about 2.5-fold significant enhancement when compared to SAHA alone or SAHA+non-targeting PIP. This would mean that SAHA+p16 has the ability to specifically induce histone H3 lysine 9 acetylation in a genomic region including the sequence recognized by SAHA+p16 because histone H3 lysine 9 and 14 acetylation is particularly known to be correlated well with induction of transcription.

### 8. Expression analysis of LARP1 gene by quantitative PCR:

HEK293 cells were used to analyze LARP1 gene expression. HEK293 cells are a cell line established by transformation of human embryonic kidney cells with the adenovirus E1 gene. The LARP1 gene includes, in its gene region, a sequence similar to the sequence of the regulatory region in the p16 gene recognized by SAHA+p16 PIP, as described above.

More specifically, the expression analysis was accomplished as follows. HEK293 cells were cultured in DMEM growth medium containing 10% fetal calf serum (FCS). During culture, the medium was replaced every two days by a culture solution containing the synthesized compound (SAHA+p16 PIP, esterified SAHA+p16 PIP or SAHA+non-targeting PIP) at a final concentration of 150 nM, and cell culture was continued for one week in the medium containing the above synthesized compound. After one week, the cells were lysed with 1 ml of TRIzol and then mixed with 200 µl of chloroform, followed by centrifugation at 12000 x g at 4°C for 10 minutes. Then, the supernatant was mixed with an equal volume of isopropanol and centrifuged at 12000 x g at 4°C for 10 minutes. Then, the pellet was washed with a 70% ethanol solution and centrifuged at 5000 x g for 5 minutes to remove the supernatant. Then, the pellet was dissolved in 100 µl water and the resulting RNA solution was applied to the column of an RNeasy kit (QIAGEN) to purify RNA. The RNA (1 µg) was used as a template to synthesize cDNA, followed by quantitative PCR. In this case, the following primers were used for amplification.
LARP 1 gene expression assay primers
LARP1 forward: 5'-TACCAACCACTGTCCCAGAG-3'(SEQ ID NO: 10)
LARP1 reverse: 5'-TGGGTTTGAACTGTGTCTTG-3' (SEQ ID NO: 11)

Likewise, for amplification of human endogenous glyceraldehyde-3-phosphate dehydrogenase (GAPDH) used as an internal standard, the following primers were used in PCR reaction.
Forward:
5'-GCACCGTCAAGGCTGAGAAC-3' (SEQ ID NO: 4),
Reverse:
5'-TGGTGAAGACGCCAGTGGA-3' (SEQ ID NO: 5).

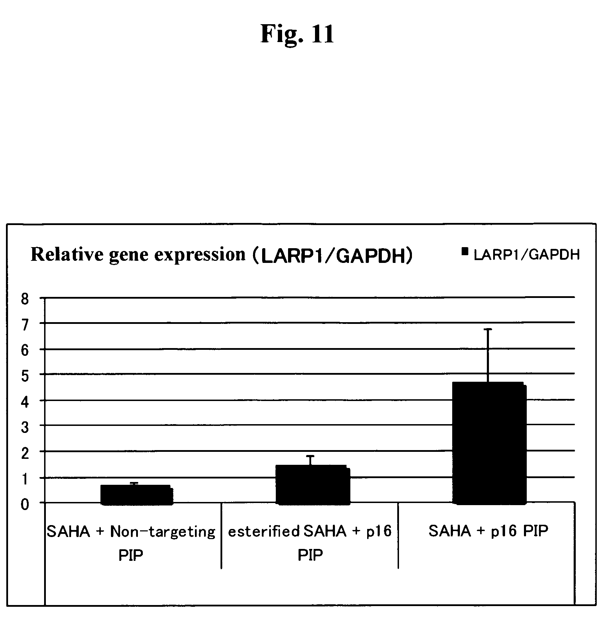

The results obtained are shown in Figure 11. As shown in Figure 11, the group receiving SAHA+p16 PIP showed increased expression of the LARP1 gene in HEK293 cells, and caused a significant increase in LARP1 gene expression when compared to esterified SAHA+p16 PIP or SAHA+non-targeting PIP.

As shown in Figure 10, SAHA+p16 PIP enhances acetylation of histone H3 lysine 9 in the genomic region of the LARP1 gene. Histone H3 lysine 9 acetylation is particularly known to be correlated with induction of gene expression.

In consideration of these results shown in Figures 10 and 11, it was predicted that SAHA+p16 PIP would also inhibit histone deacetylase in the LARP1 gene region and induce histone acetylation, thus resulting in enhanced gene expression. This suggests that SAHA+p16 PIP induced inhibition of histone deacetylase in the target genomic region to thereby activate gene expression.

### 9. Design and synthesis of conjugate (II):

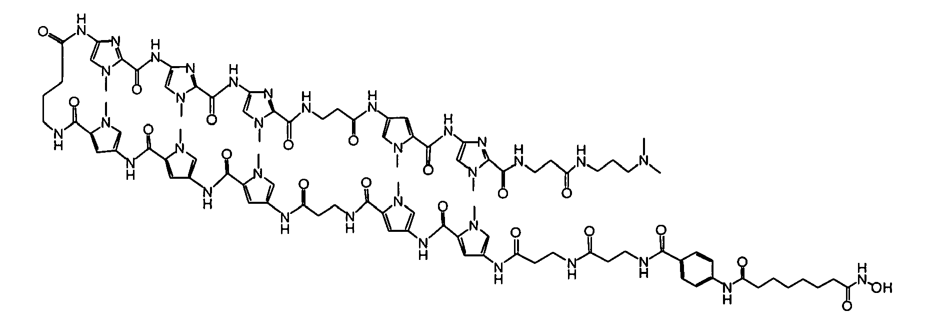

SAHA+p16NU PIP
Chemical Formula: C₉₅H₁₂₀N₃₄O₂₀
Molecular Weight: 2058.19

The above conjugate (SAHA+p16NU PIP) is a conjugate between PIP capable of recognizing the promoter region of the p 16 gene (p16NU PIP) and SAHA.

The above conjugate was designed as follows.

In the sequence of the promoter region of the p16 gene shown in Figure 1, the bold sequence (AGGGTTGAGGGGGTAGGGGGACACTTTCTAGT) found at the top, which is a linker sequence region, is located near the transcription factor binding site and hence would induce histone acetylation. For this reason, the shadowed sequence AGGGTTGA found at the top of the sequence shown in Figure 1 was used as a target and a polyamide was designed to recognize this sequence.

The above conjugate was synthesized as follows.

### Synthesis of H₂NββPyPyβPyPyPyγImImImβPyImβDp polyamide 8:

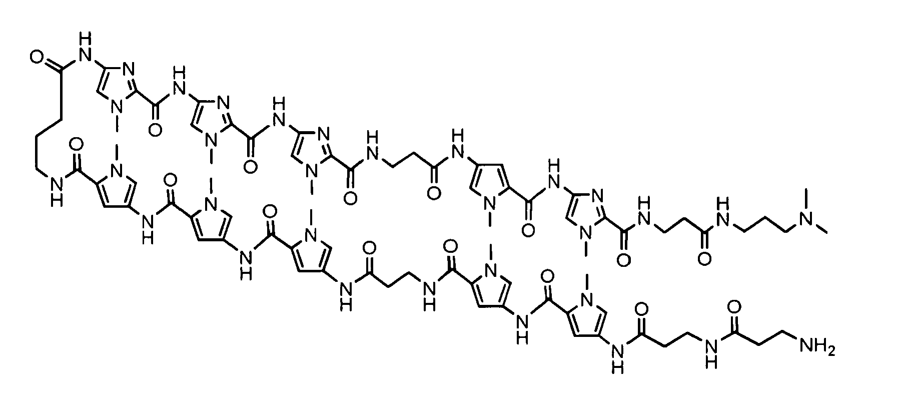

A Libra tube was charged with β-CLEAR resin (241.4 mg, 0.122 mmol), and 15 Eppendorf tubes were each charged with 1-[bis(dimethylamino)methylene]-5-chloro-1H-benzotriazolium-3-oxide hexafluorophosphate (160 mg, 0.39 mmol). Among these 15 tubes, 6 tubes were further charged with 4-(Fmoc-amino)-1-metyl-1H-pyrrole-2-carboxylic acid (145 mg, 0.4 mmol), 4 tubes were further charged with 4-(Fmoc-amino)-1-metyl-1H-imidazole-2-carboxylic acid (145 mg, 0.4 mmol), another 4 tubes were further charged with N-β-Fmoc-β-alanine (130 mg, 0.42 mmol), and the remaining one tube was further charged with N-γ-Fmoc-γ-aminobutyric acid (150 mg, 0.46 mmol). All of the samples were dried under reduced pressure. After fully drying, DMF (2 ml) was added to the Libra tube to swell the resin and then immediately distilled off. A 20% piperidine/DMF solution (3 ml) was added for deprotection to remove Fmoc protecting groups. Removal of Fmoc was confirmed by measuring UV-Vis absorption. After deprotection, the solid phase was washed with CH₂Cl₂ (5 ml), methanol (5 ml) and DMF (5 ml). Then, the first mixture of reaction reagent and HCTU prepared above was added to the Libra tube, to which DMF (5 ml) and DIEA (70 µl) as a base were then added and dissolved. The mixture was reacted under stirring at room temperature for 1 hour. After 1 hour, acetic anhydride (0.2 ml) was added and reacted for 10 minutes to protect unreacted amino terminals with acetyl groups to thereby prevent further elongation reaction. Subsequently, the solvent was distilled off and the solid phase was washed with CH₂Cl₂ (5 ml), methanol (5 ml) and DMF (5 ml), followed by Fmoc deprotection with a 20% piperidine/DMF solution (3 ml). The same treatment was then repeated until the intended sequence was obtained. The last reagent was used in the reaction and deprotected to remove Fmoc. After drying, the entire solid phase was transferred to a 10 ml recovery flask, to which N,N-dimethyl-1,3-propanediamine (1 ml) was then added and stirred at 55°C for 8 hours, followed by excision of the resulting polyamide from the solid phase. The generation of desired polyamide 8 was confirmed by HPLC (0.1% AcOH-CH₃CN, 0-100%, 20 min, 254 nm) and ESI-MS. N,N-Dimethyl-1,3-propanediamine was distilled off and the polyamide was washed with Et₂O to give 7 as a yellow brown solid, which was obtained in a crude yield of 48.0 mg (23%).

ESI-MS m/z calcd for C₈₀H₁₀₂N₃₂O₁₆⁺ [M+H]⁺ 1767.9, found 1768.0

### Synthesis of 4-(8-methoxy-8-oxooctanamido)benzoyl-H₂NββPyPyβPyPyPyγImImImβPyImβDp polyamide 22

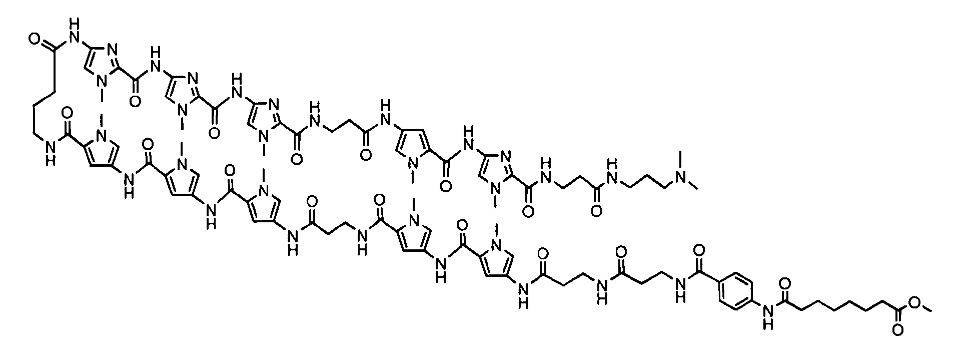

A 10 ml recovery flask was charged with 4-(8-methoxy-8-oxooctanamido)benzoic acid 5 (16.9 mg, 0.054 mmol), H₂N-ββImPyPyβImPyγImPyβImImPyβDp 8 (20.2 mg, 11.0 µmol) and FDPP (68.4 mg, 0.22 mmol). After degassing and drying, the flask was purged with Ar using a three-way stopcock. Then, dehydrated DMF (1.5 ml) and DIEA (0.15 ml) as a base were added, and the mixture was stirred at room temperature for 6 hours. The reaction was monitored by HPLC (0.1% AcOH-CH₃CN, 0-100%, 20 min, 254 nm). After confirming that H₂N-ββImPyPyβImPyγImPyβImImPyβDp 8 was completely consumed, the solvent was distilled off under reduced pressure. Et₂O (3 ml) was added to dissolve and remove soluble components, and only insoluble components were collected. The collected insoluble components were dissolved in DMF (1 ml) and purified by HPLC on a Chemcobond 5-ODS-H column (0.1% AcOH/CH₃CN 0-100%, 20 min, 360 nm) to give 22 as a white solid.

Yield: 7.0 mg (33%). ESI-MS m/z calcd for C₉₆H₁₂₁N₃₃O₂₀⁺, [M+H]⁺ 2057.2, found 2057.1.

### Synthesis of SAHA-H₂NββPyPyβPyPyPyγImImImβPyImβDp 20 (SAHA+p16Nu PIP):

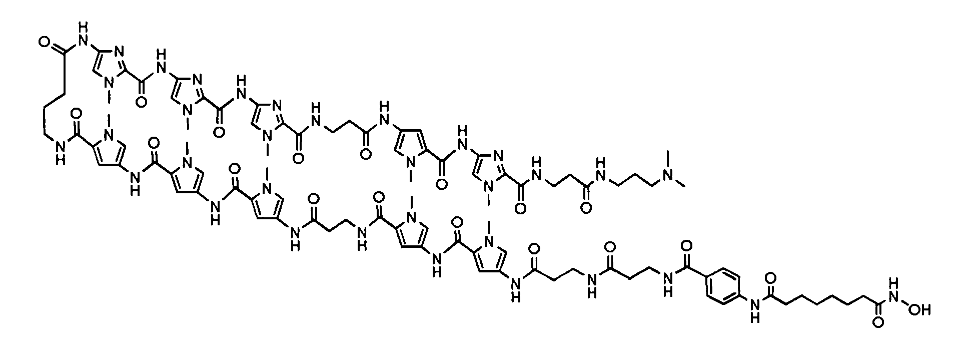

In a 10 ml recovery flask, 4-(8-methoxy-8-oxooctanamido)benzoyl-ββImPyPyβImPyγImPyβImImPyβDp 22 (7.0 mg, 3.5 µmol) was dissolved in DMF (2.0 ml) and mixed with 50% NH₂OH/H₂O (1.0 ml, 15.1 mmol), followed by stirring at room temperature for 10 hours. The reaction was monitored by HPLC (0.1% AcOH/CH₃CN 0-100%, 0-100%, 20 min, 254 nm). After confirming that 4-(8-methoxy-8-oxooctanamido)benzoyl-ββImPyPyβImPyγImPyβImImPyβDp 22 was completely consumed, the reaction was quenched with acetic acid under ice cooling and the solvent was then distilled off under reduced pressure. The resulting residue was dissolved in DMF (1 ml) and purified by HPLC on a Chemcobond 5-ODS-H column (50 mmol AF-CH₃CN, 20 min, 360 nm) to give 15 as a white solid.

Yield: 4.0 mg (49%). ¹H NMR (DMSO-D₆, 400 MHz, δ): ESI-MS m/z calcd for C₉₅H₁₂₀N₃₄O₂₀⁺. [M+H]⁺ 2058.9, found 2059.2.

### 10. Cell proliferation assay (II):

MDA-MB231 breast cancer cells (1 x 10⁵ cells) were cultured in 6 cm diameter dishes using RPMI-1640 medium containing fetal bovine serum at a final concentration of 10%. For culture, the medium was supplemented with or without each polyamide (SAHA+p16NU PIP or SAHA+non-targeting PIP) at the final concentration indicated below.
No treat: untreated group
1 µM SAHA+non-targeting PIP
1 µM SAHA+p16NU PIP
2 µM SAHA+p16NU PIP

Starting from the initiation of culture, the following operation was repeated every 24 hours. Namely, after being observed under a microscope, the cells were harvested and counted to determine the numbers of viable cells positive for trypan blue staining and dead cells negative for trypan blue staining. As a result, proliferation of the cells and their survival rate were determined.

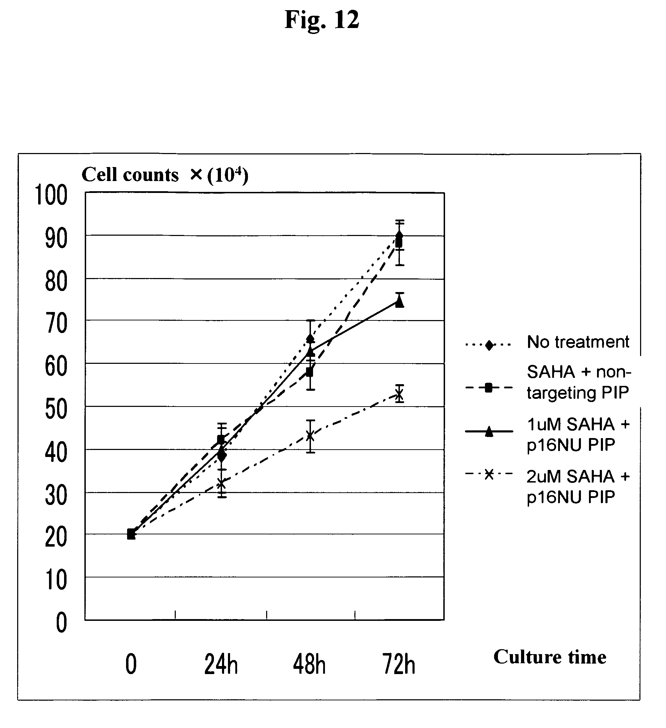

The results obtained are shown in Figure 12. In Figure 12, the vertical axis represents cell counts (x(10⁴)). As shown in Figure 12, after culture for 72 hours, cell proliferation was significantly suppressed in the group receiving 1 µM SAHA+p16NU PIP (83%) and in the group receiving 2 µM SAHA+p16NU PIP (about 60%) when compared to the untreated group or the group receiving 1 µM SAHA+non-targeting PIP. Moreover, in consideration of the fact that SAHA alone did not suppress tumor growth, as shown in Figure 3 above, it is recognized that the polyamide capable of recognizing the regulatory region in the p16 gene (SAHA+p16NU PIP) guides SAHA toward the target genomic region and further induces histone deacetylase inhibition in the target genomic region, as a result of which cell proliferation is suppressed. In cell proliferation suppression assay, the conjugates with SAHA synthesized for each polyamide p16 PIP or p16NU PIP, which was prepared for the P16 promoter region, were found to induce proliferation of cancer cell lines whose proliferation cannot be induced by SAHA alone.

### (Conjugate synthesis example 1)

### SAHA+p53 PIP:

p53 is a gene most commonly known as a tumor suppressor gene and will frequently undergo mutations and/or DNA methylation in human carcinomas. As in the case of the p16 tumor suppressor gene, compounds according to the present invention can be synthesized for the purpose of treating cancers through re-expression of the endogenous p53 gene.

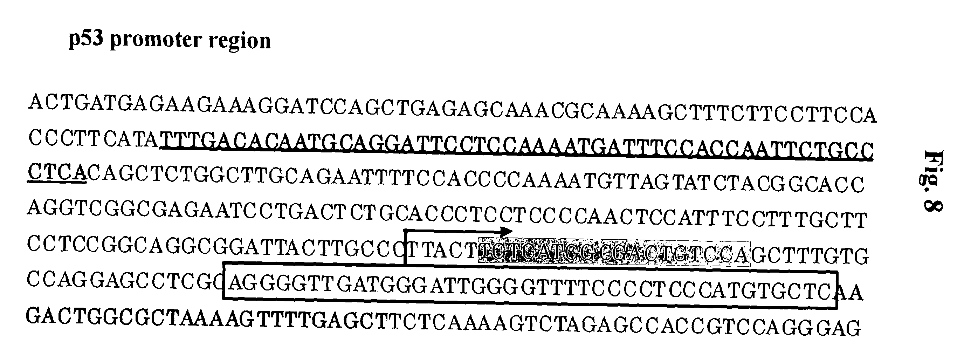

Figure 8 shows the promoter region and gene expression regulatory region of the p53 gene (SEQ ID NO: 6). In the sequence upstream of the transcription initiation site, which is located nearest to the binding region for transcription factors MycMax, MZF1, NFKB and GFI1 in the promoter region of the p53 gene, a sequence which is not included in the transcription factor-binding region but is closest to this binding region and is free from histone binding, i.e., the sequence TTTGACACAATGCAGGATTCCTCCAAAATGATTTCCACCAATTCTGCCCTCA was used as a target of PI polyamide synthesis. Namely, a polyamide was designed to recognize the underlined sequence TCCTCCA or TCCACCA (-161 to -167 or -152 to - 146) in the above sequence. Since the p53 gene is a tumor suppressor gene, the promoter region of the p53 gene is frequently methylated in cancer cells. In this case where the promoter region of the p53 gene is frequently methylated, a nucleosome structure is observed in the promoter site, to which histone proteins are bound. In this state, histones are deacetylated and DNA is condensed to generate a state where transcription factors cannot bind to the promoter region, thereby suppressing transcription of the p53 gene. The binding state of histones in the promoter region of the p53 gene can be identified by anti-histone antibody immunoprecipitation and hybridization onto a DNA oligo array. Figure 8 shows the sequence of the promoter region (-250 to +141) in the p53 gene. In the sequence shown in Figure 8, the sequence indicated with bold underline is regarded as a linker moiety which does not bind to histone proteins in cancer cells. In such a linker moiety, PI polyamide can bind to DNA, but the polyamide is highly likely not to bind to DNA in histone-binding regions because histone proteins are already bound to DNA in these regions. Moreover, in view of the facts that the boxed region is regarded as a binding site for transcription factors (which is predicted to bind to MycMax, MZF1, NFKB and GFI1), that the shadowed region is highly conserved as a regulatory region, and that this region is located in an exon downstream of the transcription initiation site, the linker sequence indicated with bold underline, i.e., TTTGACACAATGCAGGATTCCTCCAAAATGATTTCCACCAATTCTGCCCTCA, which is located upstream of the transcription initiation site and is closest to the transcription factor-binding site, would be most suitable for use as a sequence to be recognized by the polyamide. In Figure 8, it should be noted that the bold sequence located downstream of the transcription factor-binding site may affect RNA polymerase elongation and hence suppress p53 expression because it is within the transcription region. For this reason, this sequence is not defined as a region for polyamide synthesis, but this region may also be used to synthesize a compound having a similar effect on histone modification in p53. Thus, if histone acetylation can be induced in the bold underlined region, histones existing immediately downstream will be acetylated to widen their distance, thereby facilitating the binding of transcription factors to the promoter. For this reason, TCCTCCA or TCCACCA in the sequence of the bold underlined region was used as a target and the polyamide was designed to recognize this sequence. A conjugate between such a polyamide recognizing this sequence and an HDAC inhibitor would be expected to induce histone acetylation in a target-specific manner and more effectively cause a change in the nucleosome structure. Such a change in the nucleosome structure would make it possible to cause re-expression of the p53 tumor suppressor gene in cancer cells. The structural formula of SAHA+p53 PIP thus designed is shown below.

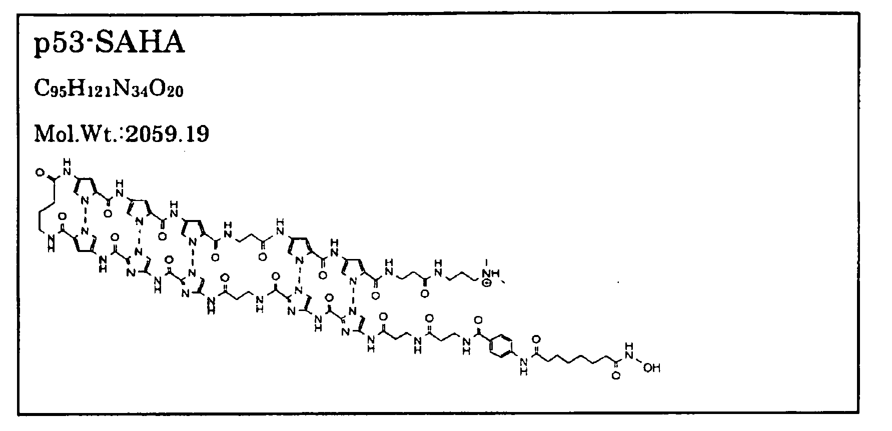

### (Conjugate synthesis example 2)

### AMI-1+MYCN PIP:

The MYCN gene is known as an oncogene and, in particular, is frequently amplified in pediatric solid tumor and neuroblastoma with poor prognosis. Some attempts have been made to treat neuroblastoma by suppression of this gene expression. Regulation of this gene expression by histone methylation not only leads to the research and development of therapy, but may also be used for therapy.

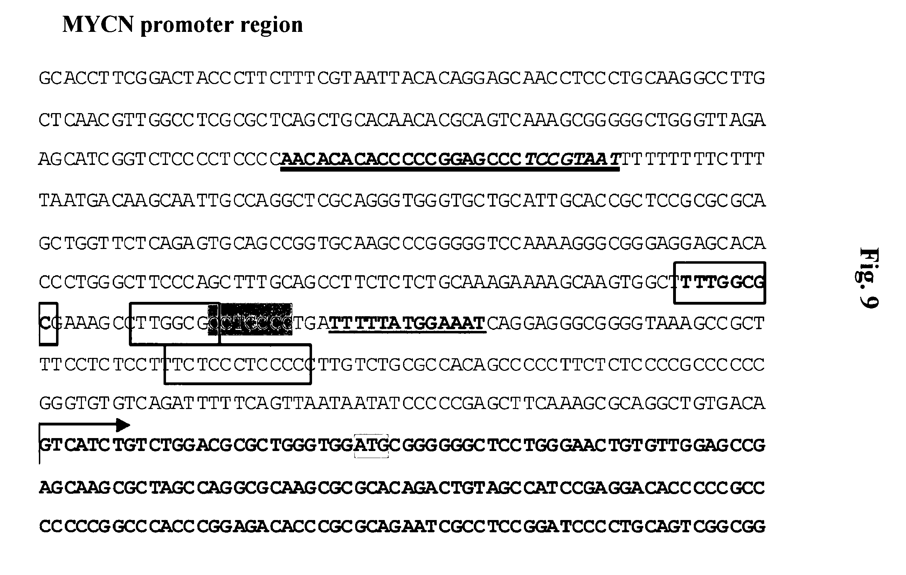

Figure 9 shows the promoter region and gene expression regulatory region of the MYCN gene (SEQ ID NO: 7). The binding regions for transcription factors E2F and MZF1 in the promoter region of the MYCN gene are regarded as weakly binding to histones. For this reason, in a region which does not affect binding to the nearest transcription factor, a sequence (-400 to -387) which weakly binds to a probe in an immunoprecipitate with histone H3K9 methylation antibody, i.e., the sequence AACACACACCCCCGGAGCCCTCCGTAAT was used as a target of PI polyamide synthesis. Namely, a polyamide was designed to recognize the underlined sequence TCCGTAAT (-380 to -387) in the above sequence. Since the MYCN gene is an oncogene, the MYCN gene is amplified and activated in its transcription, and further frequently expressed at high levels in cancer cells. When the promoter region of the MYCN gene is activated, histones would be less likely to bind to the transcription factor-binding sites in the promoter. In this state, arginine residues in histones are tended to be methylated and transcription factors bind to the promoter region to generate an activated state, thereby enhancing transcription of the MYCN gene. The binding state of histones in the promoter region of the MYCN gene can be identified by anti-histone antibody immunoprecipitation and hybridization onto a DNA oligo array. Figure 9 shows the sequence of the promoter region (-540 to +180) in the MYCN gene. In the sequence shown in Figure 9, the sequences indicated with bold underline are each regarded as a linker moiety which does not bind to histone proteins, and upstream regions thereof are expected to bind to histones. In such a linker moiety, PI polyamide can bind to DNA, but the polyamide is highly likely not to bind to DNA in histone-binding regions because histone proteins are already bound to DNA in these regions. Moreover, in view of the facts that the boxed regions are possible regions to which transcription factors bind (MZF1 is predicted to bind to two regions upstream of the E2F-binding site and a downstream region thereof) and that the shadowed region is highly conserved as a regulatory region, there is a possibility that no histone protein can be found around the non-histone binding region in the bold underlined sequence TTTTTATGGAAAT, which is close to the transcription factor binding sites. For this reason, the upstream linker sequence AACACACACCCCCGGAGCCCTCCGTAAT would be most suitable for use as a sequence to be recognized by the polyamide. Thus, if histone methylation can be inhibited in the bold underlined region, demethylation will be induced in histones existing immediately upstream to give transcriptionally inactivated histones. AMI-1, which is an inhibitor of arginine-specific methyltransferase (PRMT), is reported to inhibit methylation of arginine residues in histones, to suppress activation of nuclear receptor reporter genes in MCF-7 breast cancer cells, and to inhibit HIV-1 RT polymerase. This suggests a possibility that AMI-1-induced changes in histone modification may induce inactivation of oncogenes. For this reason, TCCGTAAT in the bold underlined region was used as a target and the polyamide was designed to recognize this sequence. A conjugate between such a polyamide recognizing this sequence and a PRMT inhibitor AMI-1 would be expected to induce histone demethylation in a target-specific manner and more effectively cause a change in the nucleosome structure. Such a change in the nucleosome structure would make it possible to suppress expression of the MYCN oncogene in cancer cells.

MYCN-AMI-1 thus designed (represented by the following structural formula) may be synthesized in a known manner (Dyes and Pigments, 1996, 32, 193).

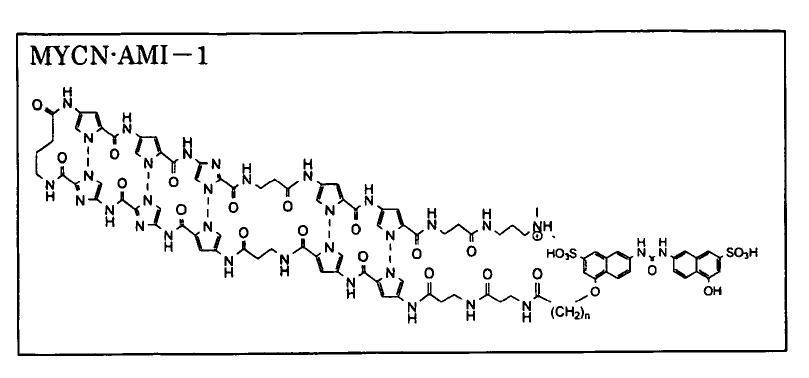

More specifically, AMI-1+MYCN PIP is synthesized as follows, starting from a histone modification regulator, J acid (2-amino-5-naphthol-7-sulfonic acid, indicated as "a" below), and the MYCN-targeting polyamide designed above. J acid and BrCH(CH₂ₙ₋₁COOH serving as an alkyl linker are reacted to synthesize a J acid derivative (indicated as "b" below), and this J acid derivative and J acid are condensed together with bis(trichloromethyl)carbonate to synthesize an AMI1 derivative (indicated as "c" below). The AMI1 derivative thus synthesized and the MYCN-targeting PIP (indicated as "d" below) are condensed together with HCTU to synthesize the desired compound AMI-1+MYCN PIP (indicated as "e" below).

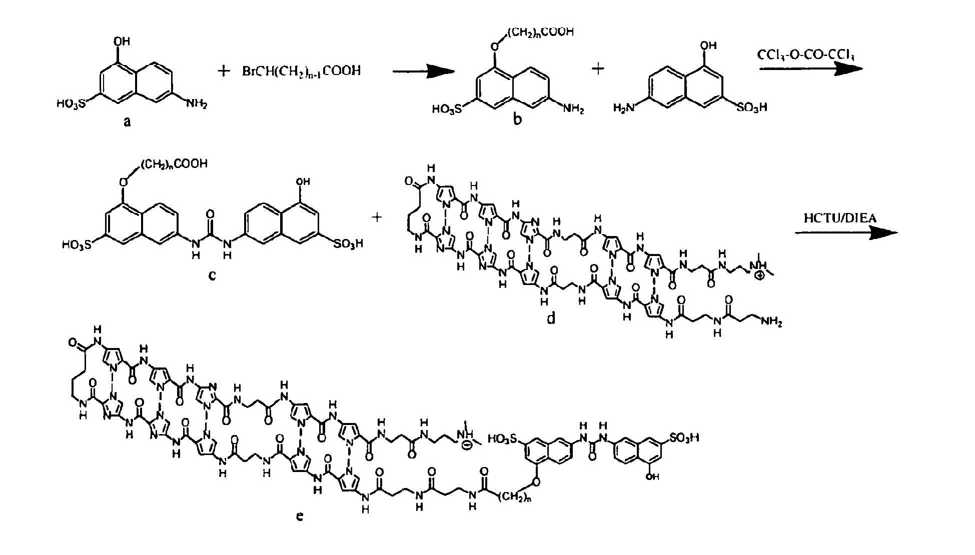

The same non-targeting PIP as used for p16 is also used, which does not recognize the sequence of the above non-histone binding linker region in the p53 promoter (i.e., which recognizes the sequence WGWCCW). The underlined linker region in the promoter shown in Figure 9 does not contain this recognition sequence.

In addition to AMI-1, any other compounds or derivatives thereof having, as their skeletal structure, J acid (indicated as "a" below), Direct Yellow 26 (indicated as "b" below), Direct Yellow 50 (indicated as "c" below), Direct Red 75 (indicated as "d" below) or suramin (indicated as "e" below), each of which is known as a PRMT inhibitor, may be used to form conjugates with PIP, and the resulting conjugates would also suppress arginine methylation in a target-specific manner and thereby inhibit expression of the MYCN oncogene.

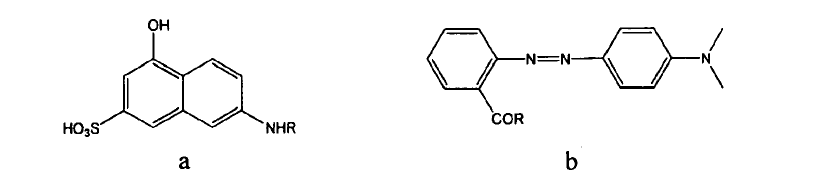

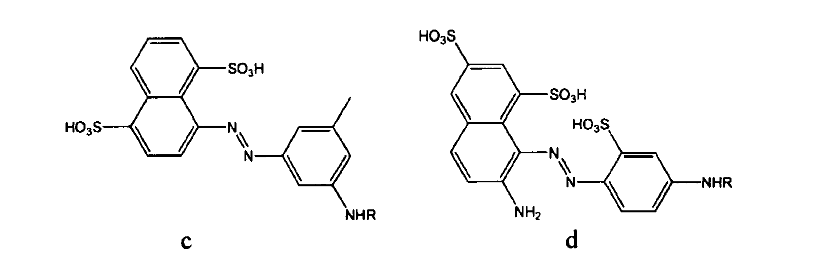

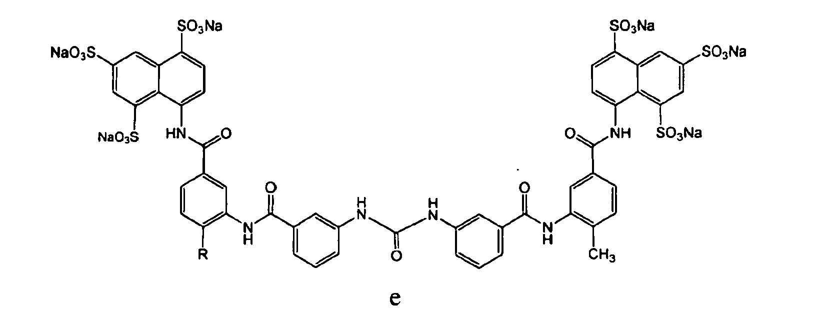

R=CONH(CH₂)ₙ-PIP

## Claims

1. A target gene-specific histone modification regulator, which comprises a conjugate between a histone modification regulator and a polyamide capable of recognizing a regulatory region in a target gene.

2. The regulator according to claim 1, wherein the histone modification regulator regulates at least one histone modification selected from the group consisting of acetylation, phosphorylation, methylation, ubiquitination, sumoylation, proline isomerization, deimination and ADP ribosylation in a genomic site-specific manner.

3. The regulator according to claim 2, wherein the histone modification regulator is a histone deacetylase inhibitor.

4. The regulator according to claim 3, wherein the histone deacetylase inhibitor is a hydroxamic derivative, a cyclic tetrapeptide, a benzamide derivative, or an aliphatic acid.

5. The regulator according to claim 4, wherein the hydroxamic derivative is suberoylanilide hydroxamic acid, suberoyl bis-hydroxamic acid or trichostatin A.

6. The regulator according to claim 4, wherein the cyclic tetrapeptide is trapoxin A or trapoxin B.

7. The regulator according to claim 4, wherein the benzamide derivative is MS-275.

8. The regulator according to claim 4, wherein the aliphatic acid is butyrate or NaB.

9. The regulator according to claim 2, wherein the histone modification regulator is a histone methyltransferase inhibitor.

10. The regulator according to claim 9, wherein the histone methyltransferase inhibitor is a compound having an aromatic ring, a compound having a heterocyclic ring, or a derivative thereof.

11. The regulator according to claim 10, wherein the compound having an aromatic ring, the compound having a heterocyclic ring, or a derivative thereof is a compound having a naphthalene compound as its skeletal structure, a compound having an azo compound as its skeletal structure, or a derivative thereof.

12. The regulator according to claim 11, wherein the compound having a naphthalene compound as its skeletal structure is AMI-1, suramin or 7-amino-4-hydroxy-2-naphthalenesulfonic acid (J acid).

13. The regulator according to claim 11, wherein the compound having an azo compound as its skeletal structure is Direct Yellow 26, Direct Yellow 50, or Direct Red 75.

14. The regulator according to any one of claims 1 to 13, wherein the target gene is a gene involved in cell regulation.

15. The regulator according to claim 14, wherein the gene involved in cell regulation is an oncogene or a tumor suppressor gene.

16. The regulator according to claim 14, wherein the gene involved in cell regulation is a gene involved in the maintenance and/or differentiation of stem cells or progenitor cells.

17. The regulator according to claim 15, wherein the oncogene is a gene for Cytoplasmic tyrosine kinase, a gene for regulatory GTPase, a gene for tyrosine kinase receptor, a gene for intracellular serine or threonine kinase or a regulatory subunit thereof, a gene for an adaptor protein in the signal transduction system, or a gene for transcription factor.

18. The regulator according to claim 15, wherein the tumor suppressor gene is p16INK4a (CDKN2A) gene, p21 (CDKN1A) gene, APC gene, RASSF1 gene, RB gene, NF1 gene, NF2 gene, p19 (CDKN2D) gene, WT1 gene, VHL gene, BRCA1 gene, BRCA2 gene, CHEK2 gene, Maspin gene, p73 gene, DPC4 (SMAD4) gene, MSH2 gene, MLH1 gene, PMS2 gene, DCC gene, PTEN gene, p57KIP2 (CDKN1C) gene, PTC gene, TSC1 gene, TSC2 gene, EXT1 gene, EXT2 gene, or p53 gene.

19. The regulator according to claim 16, wherein the gene involved in the maintenance and/or differentiation of stem cells or progenitor cells is LIF (leukaemia inhibitory factor) gene, OCT3/4 gene, NANOG gene, SOX2 gene, KLF4 gene, MYC gene, MYCN gene, or p16INK4a (CDKN2A) gene.

20. The regulator according to claim 1, wherein the conjugate is represented by the following formula (1), (2), (3), (4) or (5).

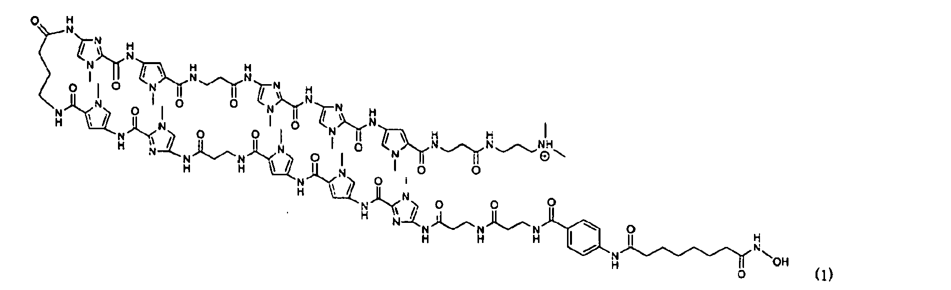

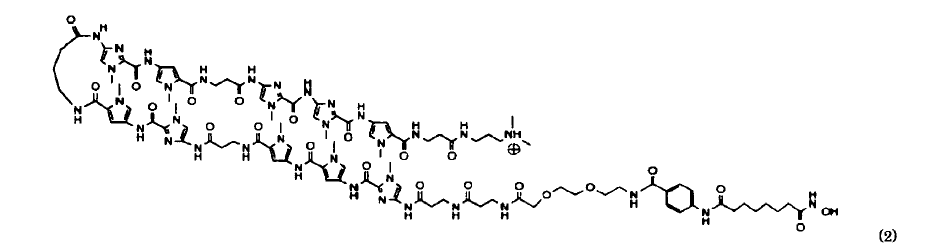

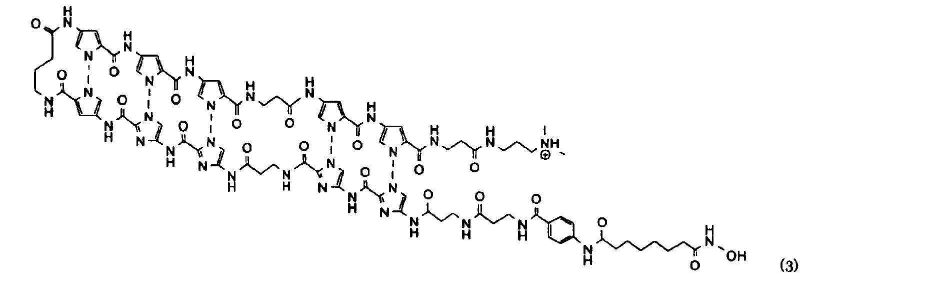

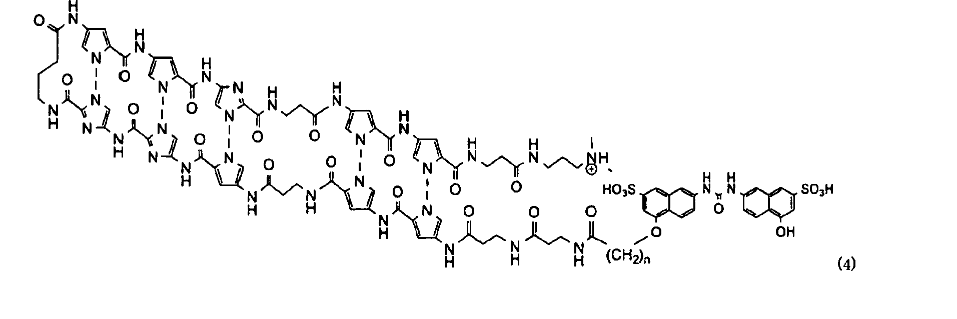

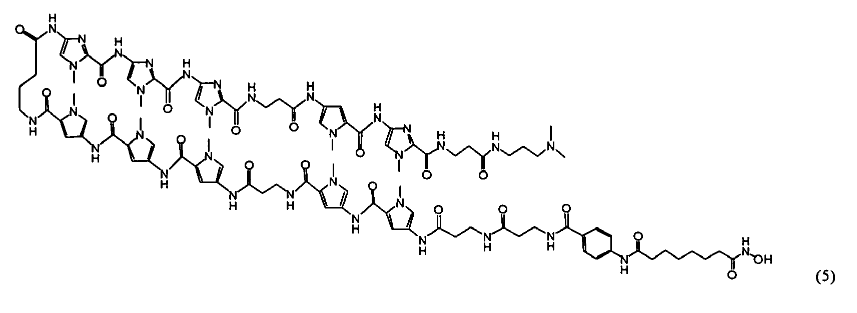

21. A pharmaceutical composition, which comprises the conjugate according to any one of claims 1 to 20.


SEQUENCE LISTING

<110>  Nihon University

<120>  Histon modification regulator which specifically binds to
       targeted gene

<130>  PCT09-0012

<150>  JP 2008-172795
<151>  2008-07-01

<160>  13

<170>  PatentIn version 3.4

<210>  1
<211>  560
<212>  DNA
<213>  Homo sapiens

<400>  1
tgaaaatcaa gggttgaggg ggtaggggga cactttctag tcgtacaggt gatttcgatt      60

ctcggtgggg ctctcacaac taggaaagaa tagttttgct ttttcttatg attaaaagaa     120

gaagccatac tttccctatg acaccaaaca ccccgattca atttggcagt taggaaggtt     180

gtatcgcgga ggaaggaaac ggggcggggg cggatttctt tttaacagag tgaacgcact     240

caaacacgcc tttgctggca ggcgggggag cgcggctggg agcagggagg ccggagggcg     300

gtgtgggggg caggtgggga ggagcccagt cctccttcct tgccaacgct ggctctggcg     360

agggctgctt ccggctggtg cccccggggg agacccaacc tggggcgact tcaggggtgc     420

cacattcgct aagtgctcgg agttaatagc acctcctccg agcactcgct cacggcgtcc     480

ccttgcctgg aaagataccg cggtccctcc agaggatttg agggacaggg tcggaggggg     540

ctcttccgcc agcaccggag                                                 560


<210>  2

<211>  20
<212>  DNA
<213>  Artificial sequence

<220>
<223>  synthetic DNA

<400>  2
ggcaccagag gcagtaacca                                                    20


<210>  3
<211>  25
<212>  DNA
<213>  Artificial sequence

<220>
<223>  synthetic DNA

<400>  3
ggaccttcgg tgactgatga tctaa                                              25


<210>  4
<211>  20
<212>  DNA
<213>  Artificial sequence

<220>
<223>  synthetic DNA

<400>  4
gcaccgtcaa ggctgagaac                                                    20


<210>  5
<211>  19
<212>  DNA
<213>  Artificial sequence

<220>
<223>  synthetic DNA

<400>  5

tggtgaagac gccagtgga 19

<210> 6
<211> 391
<212> DNA
<213> Homo sapiens

<400> 6
actgatgaga agaaaggatc cagctgagag caaacgcaaa agctttcttc cttccaccct 60

tcatatttga cacaatgcag gattcctcca aaatgatttc caccaattct gccctcacag 120

ctctggcttg cagaattttc caccccaaaa tgttagtatc tacggcacca ggtcggcgag 180

aatcctgact ctgcaccctc ctccccaact ccatttcctt tgcttcctcc ggcaggcgga 240

ttacttgccc ttacttgtca tggcgactgt ccagctttgt gccaggagcc tcgcaggggt 300

tgatgggatt ggggttttcc cctcccatgt gctcaagact ggcgctaaaa gttttgagct 360

tctcaaaagt ctagagccac cgtccaggga g 391

<210> 7
<211> 720
<212> DNA
<213> Homo sapiens

<400> 7
gcaccttcgg actacccttc tttcgtaatt acacaggagc aacctccctg caaggccttg 60

ctcaacgttg gcctcgcgct cagctgcaca acacgcagtc aaagcggggg ctgggttaga 120

agcatcggtc tcccctcccc aacacacacc cccggagccc tccgtaattt tttttctttt 180

taatgacaag caattgccag gctcgcaggg tgggtgctgc attgcaccgc tccgcgcgca 240

gctggttctc agagtgcagc cggtgcaagc ccgggggtcc aaaagggcgg gaggagcaca 300

ccctgggctt cccagctttg cagccttctc tctgcaaaga aaagcaagtg gcttttggcg 360

cgaaagcctt ggcgcctccc ctgattttta tggaaatcag gagggcgggg taaagccgct 420

ttcctctcct ttctccctcc cccttgtctg cgccacagcc cccttctctc cccgcccccc 480

gggtgtgtca gatttttcag ttaataatat cccccgagct tcaaagcgca ggctgtgaca 540

gtcatctgtc tggacgcgct gggtggatgc ggggggctcc tgggaactgt gttggagccg 600

agcaagcgct agccaggcgc aagcgcgcac agactgtagc catccgagga cacccccgcc 660

cccccggccc acccggagac acccgcgcag aatcgcctcc ggatcccctg cagtcggcgg 720

<210> 8
<211> 19
<212> DNA
<213> Artificial sequence

<220>
<223> synthetic DNA

<400> 8
ttgccaacgc tggctctgg 19

<210> 9
<211> 21
<212> DNA
<213> Artificial sequence

<220>
<223> synthetic DNA

<400> 9
caaatcctct ggagggaccg c 21

<210> 10
<211> 20
<212> DNA
<213> Artificial sequence

<220>
<223> synthetic DNA

<400> 10

taccaaccac tgtcccagag                                                    20

<210>  11
<211>  20
<212>  DNA
<213>  Artificial sequence

<220>
<223>  synthetic DNA

<400>  11
tgggtttgaa ctgtgtcttg                                                    20

<210>  12
<211>  20
<212>  DNA
<213>  Artificial sequence

<220>
<223>  synthetic DNA

<400>  12
tactagcggt tttacgggcg                                                    20

<210>  13
<211>  24
<212>  DNA
<213>  Artificial sequence

<220>
<223>  synthetic DNA

<400>  13
tcgaacagga ggagcagaga gcga                                               24